(19) Europäisches Patentamt
European Patent Office
Office européen des brevets

(11) EP 1 487 434 B1

(12) # EUROPEAN PATENT SPECIFICATION

(45) Date of publication and mention of the grant of the patent:
**09.05.2007 Bulletin 2007/19**

(21) Application number: **03713959.9**

(22) Date of filing: **06.03.2003**

(51) Int Cl.:
***A61K 31/352*** *(2006.01)* ***A61K 31/122*** *(2006.01)*
***A61K 31/37*** *(2006.01)* ***A61K 36/00*** *(2006.01)*
***A61K 45/06*** *(2006.01)* ***A61P 35/00*** *(2006.01)*

(86) International application number:
**PCT/US2003/006979**

(87) International publication number:
**WO 2003/075943 (18.09.2003 Gazette 2003/38)**

(54) **BOTANICAL EXTRACT COMPOSITIONS WITH ANTI-CANCER ACTIVITY COMPRISING ISOLIQUIRITIGENIN**

BOTANISCHER EXTRAKT MIT ANTIKREBS-AKTIVITÄT ENTHALTEND ISOLIQUIRITIGENIN

COMPOSITIONS D'EXTRAIT BOTANIQUE AYANT UNE ACTION ANTICANCEREUSE CONTENANT DU ISOLIQUIRITGENINE

(84) Designated Contracting States:
**AT BE BG CH CY CZ DE DK EE ES FI FR GB GR HU IE IT LI LU MC NL PT RO SE SI SK TR**

(30) Priority: **06.03.2002 US 362420 P**
**22.04.2002 US 374417 P**

(43) Date of publication of application:
**22.12.2004 Bulletin 2004/52**

(73) Proprietor: **The Medical Research and Education Trust**
**San Diego, CA 92101 (US)**

(72) Inventor: **CHEN, Sophie PH.D**
**Millwood, NY 10546 (US)**

(74) Representative: **Jones, Elizabeth Louise**
**Frank B. Dehn & Co.**
**St Bride's House**
**10 Salisbury Square**
**London EC4Y 8JD (GB)**

(56) References cited:
**EP-A- 0 291 151**
**EP-A- 0 633 022**
**EP-A- 0 656 213**
**EP-A- 0 742 012**
**EP-A- 0 906 761**
**EP-A- 1 029 545**
**EP-A- 1 127 572**
**EP-A- 1 374 880**
**WO-A-00/03706**
**WO-A-00/03707**
**WO-A-01/03716**
**WO-A-01/21009**
**WO-A-01/30342**
**WO-A-01/66123**
**WO-A-01/68098**
**WO-A-01/78783**
**WO-A-01/80855**
**WO-A-02/34073**
**WO-A-95/20960**
**WO-A-97/11692**
**WO-A-98/09615**
**WO-A-98/48790**
**WO-A-02/076241**
**WO-A-02/076484**
**WO-A-02/080951**
**WO-A-03/074065**
**DE-A- 10 118 999**
**FR-A- 2 658 420**
**GB-A- 1 476 016**
**GB-A- 2 346 325**
**US-A- 5 935 996**
**US-A1- 2003 035 851**

- **IKEMOTO SHINICHI ET AL: "Antitumor effects of Scutellariae radix and its components baicalein, baicalin, and wogonin on bladder cancer cell lines" UROLOGY, vol. 55, no. 6, June 2000 (2000-06), pages 951-955, XP002257001 ISSN: 0090-4295**
- **LEE WOAN-ROUH ET AL: "Wogonin and fisetin induce apoptosis in human promyeloleukemic cells, accompanied by a decrease of reactive oxygen species, and activation of caspase 3 and Ca2+-dependent endonuclease" BIOCHEMICAL PHARMACOLOGY, vol. 63, no. 2, 15 January 2002 (2002-01-15), pages 225-236, XP001162761 ISSN: 0006-2952**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



(Cont. next page)

- DATABASE WPI Week 199737, 8 July 1997 (1997-07-08) Derwent Publications Ltd., London, GB; AN 1997-399429 XP002257002 MORINO MASAYOSHI ET AL.: "Flavonoid-containing agent for suppressing synthesis of protein of HSP27 family" & JP 09 176011 A (KUREHA CHEM IND CO LTD), 8 July 1997 (1997-07-08)
- MIAO-JING LAI ET AL.: "Relative flavone bioavailability of Scutellariae Radix between traditional decoction and commercial powder preparation in humans" J. FOOD AND DRUG ANALYSIS, vol. 10, no. 2, June 2002 (2002-06), pages 75-80, XP001170316
- DATABASE WPI Week 199047 Derwent Publications Ltd., London, GB; AN 1990-352780 XP002268303 IKEGAWA TETSUDO ET AL.: "Carcinostatic assistant" & JP 02 255622 A (TSUMURA & CO), 16 October 1990 (1990-10-16)
- DATABASE WPI Week 199429, 21 June 1994 (1994-06-21) Derwent Publications Ltd., London, GB; AN 1994-238658 XP002268304 KAMATAKI TETSUYA: "UDP-glucuronyl transferase inhibitor" & JP 06 172195 A (TSUMURA & CO.), 21 June 1994 (1994-06-21)
- DATABASE WPI Week 198543, 12 September 1985 (1985-09-12) Derwent Publications Ltd., London, GB; AN 1985-266688 XP002268305 TAKAHASHI NOBUTAKA ET AL.: "Carcinostatic agent" & JP 60 178815 A (RIKAGAKU KENKYUSHO), 12 September 1985 (1985-09-12) & PATENT ABSTRACTS OF JAPAN vol. 010, no. 023 (C-325), 29 January 1986 (1986-01-29) & JP 60 178815 A (RIKAGAKU KENKYUSHO), 12 September 1985 (1985-09-12)
- MA JING ET AL: "Apoptosis induced by isoliquiritigenin in human gastric cancer MGC-803 cells" PLANTA MEDICA, vol. 67, no. 8, November 2001 (2001-11), pages 754-757, XP008026919 ISSN: 0032-0943
- DATABASE WPI Week 199409, 1 February 1994 (1994-02-01) Derwent Publications Ltd., London, GB; AN 1994-071834 XP002268306 AOKI YASUO ET AL.: "Enhancer for carcinostatic activity" & JP 06 024975 A (DAINIPPON INK & CHEMICALS), 1 February 1994 (1994-02-01)
- DATABASE WPI Week 198701, 21 November 1986 (1986-11-21) Derwent Publications Ltd., London, GB; AN 1987-003722 XP002268307 KINOSHITA MASAHIKO: "Carcinostatic adjuvant" & JP 61 263925 A (KANEBO LTD), 21 November 1986 (1986-11-21)
- KUBO M ET AL: "SCUTELLARIAE RADIX 7. ANTI-ARTHRITIC AND ANTI-INFLAMMATORY ACTIONS OF METHANOLIC EXTRACT AND FLAVONOID COMPONENTS FROM SCUTELLARIAE RADIX" CHEMICAL AND PHARMACEUTICAL BULLETIN (TOKYO), vol. 32, no. 7, 1984, pages 2724-2729, XP008026902 ISSN: 0009-2363
- DATABASE WPI Week 199626, 23 April 1996 (1996-04-23) Derwent Publications Ltd., London, GB; AN 1996-255073 XP002268308 KUMAGAI KAZUO ET AL.: "Inhibitor of matrixmetalloprotease" & JP 08 104628 A (SUMITOMO PHARMA), 23 April 1996 (1996-04-23)
- DATABASE WPI Week 200066, 5 September 2000 (2000-09-05) Derwent Publications Ltd., London, GB; AN 2000-675074 XP002268309 OKADA TADASHI ET AL.: "Oral antibacterial agent" & JP 2000 239136 A (ORIZA YUKA KK), 5 September 2000 (2000-09-05)
- DATABASE WPI Week 199247, 8 October 1992 (1992-10-08) Derwent Publications Ltd., London, GB; AN 1992-385398 XP002268310 MATSUURA MASARU: "Agents for alleviating periodontitis" & JP 04 283518 A (KIKKOMAN CORP), 8 October 1992 (1992-10-08)
- DATABASE WPI Week 200145, 24 April 2001 (2001-04-24) Derwent Publications Ltd., London, GB; AN 2001-421066 XP002268311 & JP 2001 114675 A (F)
- KUTTAN R ET AL: "POTENTIAL ANTICANCER ACTIVITY OF TURMERIC (CURCUMA LONGA)" CANCER LETTERS, NEW YORK, NY, US, vol. 29, no. 2, November 1985 (1985-11), pages 197-202, XP001120343 ISSN: 0304-3835
- DATABASE WPI Week 197803, 3 December 1977 (1977-12-03) Derwent Publications Ltd., London, GB; AN 1978-05666A XP002268312 MATSUI TOKUTAROU: "antitumor agent" & JP 52 145509 A (MATSUI TOKUTAROU), 3 December 1977 (1977-12-03)

(19) Europäisches Patentamt European Patent Office Office européen des brevets

- DATABASE BIOSIS [Online]

BIOSCIENCES INFORMATION SERVICE, PHILADELPHIA, PA, US; 1995 OHNO NAOHITO ET AL: "Resistance of highly branched (1 fwdarw 3)-beta-D-glucans to formolysis" Database accession no. PREV199598411947 XP002268301 & CHEMICAL AND PHARMACEUTICAL BULLETIN (TOKYO), vol. 43, no. 6, 1995, pages 1057-1060, ISSN: 0009-2363

- DATABASE BIOSIS [Online] BIOSCIENCES INFORMATION SERVICE, PHILADELPHIA, PA, US; 1978 HAMURO J ET AL: "SOLID PHASE ACTIVATION OF ALTERNATIVE PATHWAY OF COMPLEMENT BY BETA-1 3 GLUCANS AND ITS POSSIBLE ROLE FOR TUMOR REGRESSING ACTIVITY" Database accession no. PREV197866028521 XP002268302 & IMMUNOLOGY, vol. 34, no. 4, 1978, pages 695-706, ISSN: 0019-2805
- DATABASE PUBMED [Online] FREE RADIC. BIOL. MED. 1997; 23(2), 302-313, 1997 VAYA J. ET AL: 'Antioxidant constituents from licorice roots: isolation, structure elucidation and antioxidative capcity toward LDL oxidation'
- CAO Y. ET AL: 'Determination of liqiuiritigenin and isoliquiritigenin in Glycyrrhiza uralensis and its medicinal preparations by capillary electrophoresis with electrochemical detection' J. CHROMATOGRAPHY A vol. 1042, 2004, pages 203 - 209

EP 1 487 434 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

TECHNICAL FIELD

This application related to botanical extract compositions according to claim 9 and the use thereof in the manufacture of a medicament for treating prostate cancer.

BACKGROUND

Phytoestrogens, such as the isoflavones contained in soy products, are believed to have therapeutic potential in disease treatment and prevention. In particular, phytoestrogens are believed to be useful in the treatment of estrogen-related disorders such as, for example, osteoporosis, the symptoms of menopause, and hormone-related cancers.

It has been reported that endogenous and exogenous hormones play a role in the development of hormone-related cancers, such as breast cancer, colon cancer, lung cancer, endometrial cancer, ovarian cancer, prostate cancer, bladder cancer, testicular cancer, thyroid cancer, and bone cancer (see, for example, Henderson et al, "Hormonal carcinogenesis", *Carcinogenesis* (2000), 21(3): 427-433). Epidemiological studies have shown that consumption of a diet with high content of phytoestrogens such as those found in soy products was associated with a lower incidence of hormonal related cancers (H. Wiseman, "The therapeutic potential of phytoestrogens", *Expert. Opin. Investig. Drugs* (2000), 9(8):1829-40).

Prostate carcinoma, a hormone-related cancer, is a major health problem among men in North America and Europe (S. H. Landis et al., "Cancer Statistics, 1998", *CA Cancer J. Clin.* (1998) 48: 6-29). Chronic. enlargement of the prostate in combination with elevated prostate specific antigen (PSA) can often lead to prostate carcinoma. Every year 160,000 new cases and 39,000 deaths from the disease occur in the United States (Landis). Breast cancer, another hormone-related cancer, is also a major health problem. New invasive incidences of breast carcinoma are projected to be 192,200, with 40,200 projected deaths in 2001 according to the American Cancer Society (National Alliance of Breast Cancer Organizations News, 15(1): 2, January, 2001). Early detection and early intervention are often the key solutions to treating these diseases. Although chemotherapy is often the choice for advanced-stage breast cancer patients, for example, it is not effective for the advanced-stage prostate cancer patients. Conventional treatment methods include surgery, radiation, hormone therapy, and chemotherapy. There is a need for alternative therapeutic agents that can augment or replace existing therapies.

While the existing therapeutic agents are well-suited for their intended purpose, there remains a need for other herbal remedies for the treatment of prostate cancer.

BRIEF SUMMARY

Compositions according to claim 9 useful for treating prostate cancer; are described in detail below.

BRIEF DESCRIPTION OF THE DRAWINGS

Figure 1 shows a high performance liquid chromatogram (measured at 254 nanometers) of a multi-component botanical extract composition containing extracts of Panax pseudo-ginseng Wall, Isatis Indigotica Fort, Ganoderma lucidium Karst, Dendrathema morifolium Tzvel, Glycyrrhiza glabra L, Sculletaria bailcalensis Georgi, Rabdosia rubescens, and Serenoa repens; an arrow indicates the position of wogonin (designated "I-16-2") in the elution profile.

Figure 2 shows $^{13}C$ NMR spectra of wogonin separated from a multi-component botanical extract compositions as in Figure 1; (a) separate (DEPT) spectra for $-CH_3$, $-CH_2$ and -CH groups; (b) total $^{13}C$ NMR spectrum.

Figure 3 is a mass spectrum of wogonin separated from a multi-component botanical extract as in Figure 1, with a purity of greater than 95%.

Figure 4 is a high performance liquid chromatogram of isoliquiritigenin isolated from *Glycyrrhiza uralensis.*

Figure 5 is an absorption spectra associated with the isoliquiritigenin peak in the chromatogram of Figure 4.

Figure 6 shows $^{13}C$ NMR spectra of isoliquiritigenin separated from *Glycyrrhiza uralensis;* (a) separate (DEPT) spectra for $-CH_3$, $-CH_2$ and -CH groups; (b) total $^{13}C$ NMR spectrum.

Figure 7 is a mass spectrum of isoliquiritigenin separated from *Glycyrrhiza uralensi.,* with a purity shown to be higher than 95%.

Figure 8 is a plot of cell viability of LNCaP and DU-145 prostate cancer cells as a function of wogonin concentration.

Figure 9 is a plot of cell viability of DU-145 and LNCaP prostate cancer cells, and MCF-7 breast cancer cells, as a function of isoliquiritigenin concentration.

Figure 10 displays DNA histograms showing the effect on LNCaP cell cycle in the absence (A) and presence (B) ofwogonin at 20 micrograms/milliliter.

Figure 11 shows changes in the LNCaP cell cycle induced by wogonin and isoliquiritigenin.

Figure 12 shows changes in the DU-145 cell cycle induced by wogonin and isoliquiritigenin.

Figure 13 is a plot showing the potency of wogonin and isoliquiritigenin as ER-alpha-Luc reporter gene activation. (reference)

Figure 14 is a plot showing the potency of wogonin and isoliquiritigenin as ER-beta-Luc reporter gene activation. (reference)

Figure 15 is a plot of COX-2 inhibition as a function of isoliquiritigenin concentration.

Figure 16 is a plot of cell viability of PTX 10 ovarian cancer cells (resistant to taxol) in the presence of increasing concentrations of wogonin. (reference)

Figure 17 is a plot of cell viability of PTX 10 ovarian cancer cells in the presence of increasing concentrations of isoliquiritigenin. (reference)

DETAILED DESCRIPTION OF THE PREFERRED EMBODIMENT

Disclosed herein are compositions according to claim 9 and uses thereof for treating prostrate cancer in a human in need of such treatment. As used herein, a human in need of prostate cancer treatment may be a human diagnosed with prostate cancer, or a human wanting to prevent or delay the onset of prostrate cancer, for example, a human with a family history of prostate cancer. The treatment of estrogen related disorders and cancers other than prostrate cancer e.g. breast cancer, endometrial cancer, colon cancer, lung cancer, bladder cancer, testicular cancer, ovarian cancer, thyroid cancer, or bone cancer is also disclosed. A human in need of treatment for an estrogen-related disorder may be a human diagnosed with an estrogen-related disorder such as, for example, osteoporosis or the symptoms of menopause, or a human wanting to prevent or delay the onset of an estrogen related disorder.
The treatment comprises administering a therapeutically effective amount of a composition comprising a phytoestrogen selected from the group consisting of isoliquiritigenin, its pharmaceutically acceptable salts or glycoside esters, and combinations comprising one or more of the foregoing compounds according to claims 1 and 9. As used herein, a phytoestrogen is a plant-derived compound or its metabolite that can mimic the action or modulate the binding, metabolism, or production of endogenous estrogens in the body.

As stated previously, herbal remedies have been used in the treatment of cancer. *Scutellaria baicalensis,* for example, is a source of wogonin (Y. Y. Zhang et al., "Comparative study of Scutellaria planipes and Scutellaria baicalensis", *Biomed. Chromatogra.* (1998), 12: 31-3), and *Glycyrrhiza uralensis* and *Glycyrrhiza glabra* are sources of isoliquiritigenin (H. Hayashi H. et al., "Seasonal variation of glycyrrhizain and isoliquiritgenin glycosides in the root of glycyrrhiza glabra L", *Biol. Pharm. Bull.* (1998) 21: 987-9).

Coumestrol is a phytoestrogen found in alfalfa and red clover that is known to exhibit phytoestrogenic activity (see, for example, U.S. Patent Application Publication No. 20010044431 A1 to Rodriguez).

Wogonin has been reported to be a strong anti-inflammation agent due to its inhibitory activity against cyclooxygenase 2 (COX-2) directly and against gene expression of inducible COX-2 and nitric oxide synthase (see, for example, Y. S. Chi et al., "Effect of wogonin, a plant flavone from Scutellaria radix, on the expression of cyclooxygenase-2 and the induction of inducible nitric oxide synthase and the induction of inducible nitric oxide synthase in inhibitors and lipopolysaccharide-treated RAW 264.7 cells", *Biochem. Pharmacol.* (2001), 61(11): 1417-27). However, the present inventor is aware of no reports of wogonin exhibiting estrogenic activity.

Isoliquiritigenin has been reported to possess estrogen-like activity (see, for example, S. Tamir "Estrogen-like

activity of glabrene and other constituents isolated from licorice root", *J. Steroid Biochem. Mol. Biol.* (2001), 78(3): 291-8). However, the present inventor is aware of no report that isoliquiritigenin is an inhibitor for COX-2 activity.

Recent studies have revealed the importance of COX-2 inhibitors as cancer therapeutic agents (see, for example, A. Kirschenbaum et al., "The role of cyclocxygenase-2 in prostate cancer" *Urology* (2001), 58(2 suppl. 1): 127-131; and E. T. Hawk et al., "COX-2 in Cancer-A Player That's Defining the Rules" *J. Natl. Cancer Inst.* (2002), 94(8): 545-546). The inhibitor blocks the angiogenesis of cancer and reduced the cancer metastasis (see, for example, E. Fosslien "Review: Molecular pathology of cyclooxygenas-2 in cancer-induced angiogenesis", *Ann. Clin. Lab Sci.* (2001), 31(4): 325-348).

JP-A-60178815 discloses the use of a pharmaceutical composition containing 0.3-15% of, amongst others, isoliquiritigenin against animal tumor cells.

WO-A-9809615, discloses a herbal composition for the treatment of prostate cancer containing *Isatis indigotica Fort, Panax pseudo-ginseng Wall, Ganoderma lucidum Karst, Scutellaria baicalensis Georgi, Dendranthema morifolium Tzvel, Glycyrrhiza glabra, Rabdosia rubescens and Serenca repens,* and optionally further cytotoxic agents.

EP-A-0656213 discloses compositions containing, amongst others, phloretin in hyaluronic acid as a penetration enhancer combined with other neoplastic agents. Case XIA discloses a combination of methotrexate, phloretin and alpha II interferon, and case XIB discloses the combination of methyl CCNU, carboplatin and methotrexate, phloretin and immune stimulating agents.

The present work demonstrates potent activity of wogonin and isoliquiritigenin to activate estrogen receptor-alpha and -beta and trigger biochemical reactions in cancer cells. The COX-2 inhibitory activity of isoliquiritigenin is also demonstrated. Suppressive effects of both compounds on cancer cell proliferation are also demonstrated. The cytotoxicity of wogonin and isoliquiritigenin toward cancer cells may either be dependent or independent of estrogen receptors.

The composition according to claim 9 and as used in accordance with claim 1 may may further comprise wogonin. As used herein, the term wogonin refers to CAS Reg. No. 632-85-9, also known as 5,7-dihydroxy-8-methoxy-flavone, and its pharmaceutically acceptable salts or esters, its selectively substituted analogs, as shown below, an extract from a plant of the *Scutellaria* family, or a combination comprising one or more of the foregoing compounds.

An ester of wogonin is preferably a glycoside of wogonin. There is no particular limit on the monosaccharide or polysaccharide used to form the glycoside of wogonin. Suitable monosaccharides sugars include glucose, glucuronic acid, mannose, fructose, galactose, xylose, rutinose or rhamnose, and combinations comprising one or more of the foregoing monosaccharides. Suitable polysaccharides include dimers, trimers, oligomers, and polymers formed from one or more of the above monosaccharides.

Wogonin analogs include, for example, genistein, biochanin, prunetin, scutellarein, daidzin, luteolin, apigenin, acacetin, 3,6,4-trihydoxylflavone, 7,3-dihydroxy-4,1-dimethoxy-isoflavone, 3R-2',3'-dihydoxy-7,4-dimethoxy-isoflavone, or a combination comprising one or more of the foregoing wogonin analogs.

Wogonin can also be in the form of an extract from a plant of the *Scutellaria* family such as, for example *Scutellaria baicalensis,* or a combination comprising one or more of the foregoing compounds.

The wogonin can comprise a selectively substituted analog of wogonin having the formula

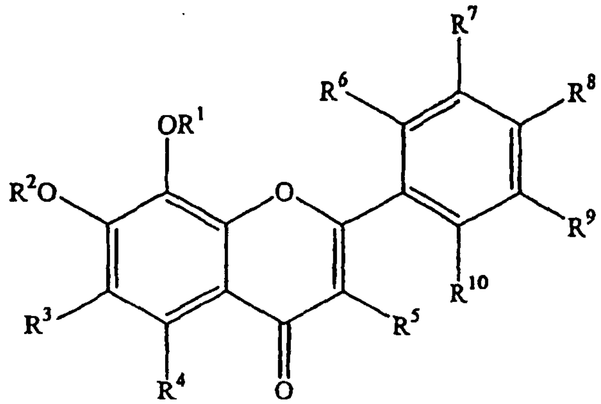

wherein $R^1$ is $C_1$-$C_6$ alkyl (preferably methyl); $R^2$ is hydrogen, $C_1$-$C_6$ alkyl, or $C_2$-$C_6$ acyl (preferably hydrogen); and $R^3$-$R^{10}$ are independently hydrogen, hydroxy, $C_1$-$C_6$ alkyl, $C_1$-$C_6$ alkoxy, or $C_2$-$C_6$ acyl (preferably hydrogen or hydroxy, more preferably hydrogen), with the proviso that at least four of $R^3$-$R^{10}$ are hydrogen. In a preferred embodiment, $R^1$ is methyl, $R^2$ is hydrogen, and $R^3$-$R^{10}$ are independently hydrogen, methyl, ethyl, methoxy, ethoxy, acetyl, or propionyl, with the proviso that at least five of $R^3$-$R^{10}$ are hydrogen.

Methods for synthesizing or isolating wogonin, its pharniaceutically acceptable salts or esters, its selectively substituted analogs, are known in the art. See, for example, International Patent Application No. WO01051482 A1 to Wallace et al; P. Rivaille et al., *C.R. Acad. Sci., Paris, Ser. C* (1969), 268(2): 2213-16; M. -C. Lin et al., *J. Chromatogr, A* (1999), 830(2): 387-395; Y. -C. Chen et al., *Biochem. Pharmacal.* (2001), 61(11): 1417-1427.

When wogonin is present, the wogonin comprises greater than or equal to 0.5 weight percent, more preferably greater than or equal to, 1 weight percent, still more preferably greater than or equal to 2 weight percent, even more preferably greater than or equal to 5 weight percent, even more preferably greater than or equal to 10 weight percent, still more preferably greater than or equal to 20 weight percent of the total weight of the composition. Compositions containing as much as 50 weight percent of wogonin are contemplated.

The phytoestrogen according to claim 9 comprises isoliquiritigenin. As used herein isoliquintigenin refers to CAS Reg. No. 961-29-5; also known as 2',4,4'-trihydroxychalcone, a pharmaceutically acceptable salt or glycoside ester of isoliquiritigenin an extract of *Glycyrrhiza uralensis* or *Glycyrrhiza glabra,* or a combination comprising one or more of the foregoing compounds.

An ester of isoliquiritigenin is a glycoside of isoliquiritigenin. There is no particular limit on the monosacharide or polysaccharide used to form the glycoside of isoliquiritigenin. Suitable monosaccharides sugars include glucose, glucuronic acid, mannose, fructose, galactose, xylose, rutinose, rhamnose, and combinations comprising one or more of the foregoing monosaccharides. Suitable polysaccharides include dimers, trimers, oligomers, and polymers formed from one or more of the above monosaccharides.

The use of an isoliquiritigenin analog for example, phloretin, 4,2,4'-trihydroxychalcone, is also disclosed.

An extract of *Glycyrrhiza uralensis* or *Glycyrrhiza glabra* is a source of isoliquiritigenin, a pharmaceutically acceptable salt or glycoside ester of isoliquiritigenin or a combination comprising one or more of the foregoing compounds.

A selectively substituted analog of isoliquiritigenin has the formula

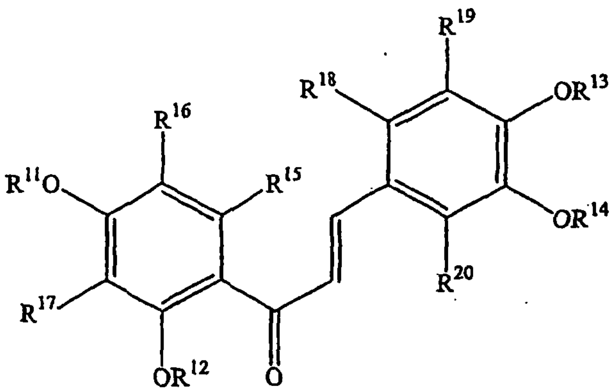

wherein $R^{11}$-$R^{14}$ are independently hydrogen or $C_1$-$C_6$ alkyl (preferably hydrogen); and $R^{15}$-$R^{20}$ are independently hydrogen, $C_1$-$C_6$ alkyl, $C_1$-$C_6$ alkoxy, or $C_2$-$C_6$ acyl (preferably hydrogen), with the proviso that at least three of $R^{15}$-$R^{20}$ are hydrogen. In a preferred embodiment, $R^{11}$-$R^{14}$ are hydrogen and $R^{15}$-$R^{20}$ are independently hydrogen, methyl, ethyl, methoxy, ethoxy, acetyl, or propionyl, with the proviso that at least four of $R^{15}$-$R^{20}$ are hydrogen.

Methods for synthesizing or isolating isoliquiritigenin, its pharmaceutically acceptable salts or glycoside esters, its selectively substituted analogs, are known in the art. See, for example, S. K. Srivastava et al., *Indian J. Chem., Sect. B* (1981), 20B(4): 347-8; and F. A. Macias et al., *Phytochemistry* (1998), 50(1): 35-46.

The isoliquiritigenin comprises greater than 0.5 weight percent, more preferably greater than or equal to 1 weight percent, still more preferably greater than or equal to 2 weight percent, even more preferably greater than or equal to 5 weight percent, even more preferably greater than or equal to 10 weight percent, still more preferably greater than or equal to 20 weight percent of the total weight of the composition. Compositions containing as much as 50 weight percent of isoliquiritigenin contemplated.

The phytoestrogen according to claim 1 or composition according to claim 9 may further comprise coumestrol. As used herein, coumestrol refers to CAS Reg. No. 479-13-0, also known as 3,9-dihydroxy-6H-benzofuro[3,2-c][1]benzopyran-6-one, a pharmaceutically acceptable salt or ester of coumestrol, a selectively substituted analog of coumestrol, an extract of *Taraxacum mongolicum,* alfalfa sprout (*Medicago sativa*), broccoli (*Brassica oleracea*), *Eclipta prostrata,* or a combination comprising one or more of the foregoing compounds.

An ester of coumestrol is preferably a glycoside of coumestrol. There is no particular limit on the monosaccharide or polysaccharide used to form the glycoside of coumestrol. Suitable monosaccharides sugars include glucose, glucuronic

acid, mannose, fructose, galactose, xylose, rutinose, rhamnose, and combinations comprising at least one of the foregoing monosaccharides. Suitable polysaccharides include dimers, trimers, oligomers, and polymers formed from one or more of the above monosaccharides.

A coumestrol analog includes 4-ethyl-7-hydroxy-3-(p-methoxyphenyl)-2H-1-benzopyran-2-one (wedelolactone).

The coumestrol may comprise an extract of *Taraxacum mongolicum,* alfafa sprout (*Medicago sativa*), broccoli (*Brassica oleracea*), *Eclipta prostrata* as a source of coumestrol, a pharmaceutically acceptable salt or ester of coumestrol, a selectively substituted analog of coumestrol, or a combination comprising one or more of the foregoing compounds.

A selectively substituted analog of coumestrol has the formula

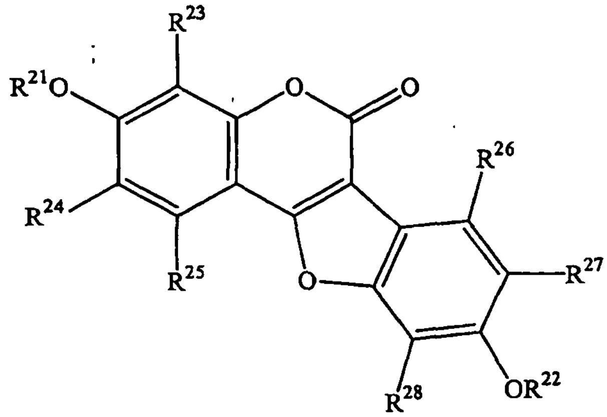

wherein $R^{21}$ and $R^{22}$ are independently hydrogen or $C_1$-$C_6$ alkyl (preferably hydrogen); and $R^{23}$-$R^{28}$ are independently hydrogen, $C_1$-$C_6$ alkyl, $C_1$-$C_6$ alkoxy, or $C_2$-$C_6$ acyl (preferably hydrogen), with the proviso that at least three of $R^{23}$-$R^{28}$ are hydrogen. In a preferred embodiment, $R^{21}$ and $R^{22}$ are hydrogen; and $R^{23}$-$R^{28}$ are independently hydrogen, methyl, ethyl, methoxy, ethoxy, acetyl, or propionyl, with the proviso that at least four of $R^{23}$-$R^{28}$ are hydrogen.

Methods for synthesizing or isolating coumestrol, its pharmaceutically acceptable salts or esters, its selectively substituted analogs, are known in the art. See, for example, P. M. Dewick et al., *J. Chem. Soc. D* (1969), 9: 466-7; T. Kappe et al., Z. *Naturforsch., Teil B* (1974), 29(3-4): 292-3; R. Laschober et al., *Synthesis* (1990), 5: 387-8; F. A. Macias et al., *Phytochemistry* (1998), 50(1): 35-46; K. Hiroya et al., *Perkin* 1 (2000), 24: 4339-4346; G. A. Kraus et al., *Journal of Organic Chemistry* (2000), 65(18): 5644-5646; and M. Okada et al., *Planta Med.* (2000), 66(6): 572-575.

When present, the coumestrol comprises greater than or equal to 0.5 weight percent, more preferably greater than or equal to 1 weight percent, still more preferably greater than or equal to 2 weight percent, even more preferably greater than or equal to 5 weight percent, even more preferably greater than or equal to 10 weight percent, still more preferably greater than or equal to 20 weight percent of the total weight of the composition. Compositions containing as much as 50 weight percent of coumestrol are contemplated.

Supplementary active anti-cancer ingredients are incorporated into the compositions and preparations according to claim 9 used according to claim 1. Administration of isoliquiritigenin optionally in admixture with wogonin and/or coumestrol in combination with other anti-cancer agents is expected to stimulate anti-cancer activity.

The composition according to claim 9 and as used in claim 1 comprises greater than 0.5 weight percent of a phytoestrogen selected from isoliquiritigenin. Wogonin and/or coumestrol may also be present, and at least one anti-cancer agent. In such compositions, the isoliquiritigenin, may be present in an amount of 0.5 to 50 weight percent of the total weight of active ingredients in the composition. Within this range, the amount may be greater than or equal to 1, 2, 5, or 10 weight percent. Also within this range, the amount may be up to 40, 30, or 20 weight percent. The above weight percents are based on the total weight of active ingredients in the composition,

The anti-cancer agents are selected from the group consisting of oridonin, indirubin, taxol, cis-platin, camptothecan, vincristine, monocrotaline, Maytansine, homoharringtonine, colchicine, irisquinone A, irisquinone B, irisquinone C, acronycine, matrin, oxymatrin, curcumin, paricine, pariphyllin, and combinations comprising one or more of the foregoing anti-cancer agents. Preferred anti-cancer agents include oridonin.

Also disclosed are alternative anti-cancer agents including an extract of a plant selected from *Rabdosia rubescens, Panax pseudo-ginseng Wall, Ganoderma lucidum Karst, Scutellaria baicalensis Georgi, Glycine max, Curcuma longa,* and combinations comprising one or more of the foregoing plants. An extract of *Rabdosia rubescens* may comprise oridonin; an extract of *Humulus lupulus* may comprise lupulone; an extract of *Panax pseudo-ginseng Wall* may comprise

a gensenoside; an extract of *Scutellaria baicalensis Georgi* may comprise baicalin; an extract of *Glycine max* may comprise a soy flavonoid, a soy isoflavonoid, or both; and an extract of *Curcuma longa* may comprise curcumin.

These alternative anti-cancer agents may comprise, 1 to 10 parts by weight of an extract of *Rabdosia rubescens;* 10 to 40 parts by weight of an extract of *Panax pseudo-ginseng Wall;* 100 to 500 parts by weight of an extract of *Ganoderma lucidum Karst;* 10 to 100 parts by weight of an extract of *Scutellaria baicalensis Georgi;* 10 to 100 parts by weight of an extract of *Glycine max*; and 10 to 100 parts by weight of an extract of *Curcuma longa.*

These alternative anti-cancer agents may comprise, an extract of *Humulus lupulus;* and an extract of a plant selected from the group consisting *of Panax pseudo-ginseng Wall, Ganoderma lucidum Karst, Scutellaria baicalensis Georgi, Glycine max, Curcuma longa,* and combinations comprising one or more of the foregoing plants.

These alternative anti-cancer agents may comprise, 10 parts by weight of an extract of *Humulus lupulus*; 10 to 40 parts by weight of an extract of *Panax pseudo-ginseng Wall*; 100 to 500 parts by weight of an extract of *Ganoderma lucidum Karst*; 10 to 100 parts by weight of an extract of *Scutellaria baicalensis Georgi*; .10 to 100 parts by weight of an extract of *Glycine max*; and 10 to 100 parts by weight of an extract of *Curcuma longa.*

The anti-cancer agent may be present at 1 to 90 weight percent of the total weight of active ingredients in the composition. Within this range, the anti-cancer agent amount may be greater than or equal to 2, 5, or 10 weight percent. Also within this range, the anti-cancer agent amount may be up to 80, 70, 50, or 25 weight percent.

In addition to an anti-cancer agent, the composition according to claim 9 and as used in claim 1 further comprises an immune stimulant, selected from the group consisting of ginsenosides, ferulic acid, mannan, synanthrin, eleutheroside A, eleutheroside B, eleutheroside C, eleutheroside D, eleutheroside E, gynoside, beta-pachyman, inulin, glycoproteins, interferons, gamma-globulins, extracts of *Ganoderma lucidum,* and combinations comprising one or more of the foregoing immune stimulants. Suitable immune stimulants further include, extracts of *Ganoderma lucidum, Coriolus versicolor, Poria cocos*, and combinations comprising one or more of the foregoing extracts. Preferred immune stimulants include beta-pachyman.

The immune stimulant is employed at 1 to 90 weight percent of the total weight of active ingredients in the composition. Within this range, the immune stimulant amount may be greater than or equal to 2, 5, 10, 20, or 50 weight percent. Also within this range, the immune stimulant amount may be up to 80, 70, or 60 weight percent.

In a preferred embodiment, the composition comprises: greater than 0.5 weight percent of a phytoestrogen selected from isoliquiritigenin ;an anti-cancer agent selected from oridonin, colchicine, vincristine, camptothecan, maytansine, taxol, and combinations comprising one or more the foregoing anti-cancer agents; and an immune stimulant selected from ginsenosides, mannan, synanthrin, eleutheroside A, eleutheroside B, eleutheroside C, eleutheroside D, eleutheroside E, gynosides, beta-pachyman, interferon, and combinations comprising one or more of the foregoing immune stimulants. In this embodiment, the composition preferably comprises: 1 to 40 weight percent isoliquiritigenin. (wogonin and/or coumestrol may also be present such that there is 1 to 40 weight percent of a combination comprising isoliquiritigenin with wogonin and/or coumestrol); 0.05 to 5 weight percent of a compound selected from oridonin, camptothecan, vincristine, Indirubin, colchicine, ginsenosides, and combinations comprising one or more of the foregoing compounds; and 10 to 98 weight percent of a compound selected from beta-pachyman, mannan, synanthrin, gynosides, and combinations comprising one or more of the foregoing compounds; wherein all weight percents are based on the total weight of the composition.

In another preferred embodiment, the composition according to claim 9 and as used according to claim 1 comprises isoliquiritigenin, optionally in combination with wogonin and/or coumestrol; oridonin; and beta-pachyman. In this embodiment, the composition preferably comprises: 1 to 30 weight percent of isoliquiritigenin (wogonin and/or coumestrol may also be present such that there is 1 to 30 weight percent of a combination of isoliquiritigenin with wogonin and/or coumestrol); 0.1 to 5 weight percent of oridonin; and 20 to 90 weight percent of beta-pachyman; wherein all weight percents are based on the total weight of the composition.

As the composition may be defined as comprising multiple components, it will be understood that each component is chemically distinct, particularly in the instance that a single chemical compound may satisfy the definition of more than one component. Wogonin, isoliquiritigenin, coumestrol, their pharmaceutically acceptable salts or esters, or their selectively substituted analogs, as defined herein before, may be isolated from natural sources or synthesized according to known methods, as described above. Purities of these compounds, as employed in the composition, may vary according to their method of isolation or synthesis, but purities of 5 percent to greater than 99 percent may be suitable for use in the composition.

The phytoestrogens may be in the form of a pharmaceutically acceptable composition. Methods for the formulation of pharmaceutically acceptable compositions are generally known. The subject pharmaceutical formulations may comprise one or more non-biologically active compounds, i.e., excipients, such as stabilizers (to promote long term storage), emulsifiers, binding agents, thickening agents, salts, preservatives, depending on the route of administration.

For oral administration, the wogonin, isoliquiritigenin, coumestrol, their pharmaceutically acceptable salts or esters, their selectively substituted analogs, or combinations comprising one or more of the foregoing may be administered with an inert diluent or with an assimilable edible carrier, or incorporated directly with the food of the diet. The formulations

may be incorporated with excipients and used in the form of ingestible tablets, buccal tablets, troches, capsules, elixirs, suspension syrups or wafers. The tablets, troches, pills or capsules may also contain the following: a binder, such as gum tragacanth, acacia, cornstarch, or gelatin; excipients, such as dicalcium phosphate; a disintegrating agent such as corn starch, potato starch or alginic acid; a lubricant such as magnesium stearate; and a sweetening agents, such as sucrose, lactose or saccharin; a flavoring agent such as peppermint or oil of wintergreen. When the dosage unit is a capsule, it may contain, in addition to materials of the above type, a liquid carrier. Various other materials may also be present as coatings or to otherwise modify the physical form of the dosage unit. A syrup or elixir may contain sucrose as a sweetening agent, methyl and propylparabens as preservatives, a dye and flavoring such as cherry or orange flavor. Such additional materials should be substantially non-toxic in the amounts employed. Furthermore, the active agents may be incorporated into sustained-release preparations and formulations. Formulations for parenteral administration may include sterile aqueous solutions or dispersions, and sterile powders for the extemporaneous preparation of sterile, injectable solutions or dispersions. The solutions or dispersions may also contain buffers, diluents, and other suitable additives, and may be designed to promote the cellular uptake of the active agents in the composition, e.g., liposomes. Sterile injectable solutions are prepared by incorporating the active compounds in the required amount in the appropriate solvent with one or more of the various other ingredients described above, followed by sterilization. Dispersions may generally be prepared by incorporating the various sterilized active ingredients into a sterile vehicle that contains the basic dispersion medium and the required other ingredients from those listed above. In the case of sterile powders used to prepare sterile, injectable solutions, the preferred methods of preparation are vacuum-drying and freeze-drying techniques which yield a powder of the active ingredient plus any additional desired ingredient from previously sterile-filtered solutions. Pharmaceutical formulations for topical administration may be especially useful for localized treatment. Formulations for topical treatment include ointments, sprays, gels, suspensions, lotions or creams. Formulations for topical administration may include known carrier materials such as isopropanol, glycerol, paraffin, stearyl alcohol or polyethylene glycol. The pharmaceutically acceptable carrier may also include a known chemical absorption promoter. Absorption promoters include, for example, dimethylacetamide (U.S. Pat. No. 3,472,931 to Stoughton), trichloroethanol or trifluoroethanol (U.S. Pat. No. 3,891,757 to Higuchi), certain alcohols and mixtures thereof (British Patent Nos. 1,001,949 to Meyer and 1,464,975 to Astra Lakemedel). Except insofar as any conventional media or agent is incompatible with the therapeutic active ingredients, its use in the therapeutic compositions and preparations is contemplated.

The composition may, optionally, be in an ingestible form, preferably a powder, a capsule, or a tablet. Alternatively, the composition may be in the form of a suppository.

The pharmaceutical compositions described preferably contain 0.5% to 100% by weight of active agent. Within this range, the compositions and preparation may preferably comprise the active agent in an amount of at least 1, 2, 5, 10, or 20 weight percent. Also within this range, the composition may preferably comprise the active agent in an amount of up to 90, 80, 70, 60, or 50 weight percent. The amount of active compounds in such pharmaceutically useful compositions and preparations is such that a suitable dosage will be obtained. Another embodiment is the use of a composition according to claim 1 for the manufacture of a medicament for the treatment of a human having prostate cancer. Also disclosed is the use of said composition for the manufacture of a medicament for treating other cancers, e.g. an estrogen-related cancer, or other estrogen-related disorder.

The terms "treating" and "treatment" as used herein refer to reduction in seventy and/or frequency of symptoms, elimination of symptoms and/or underlying cause, prevention of the occurrence of symptoms and/or their underlying cause, and improvement or remediaton of damage. Thus, for example, the present method of "treating" prostate cancer, as the term is used herein, encompasses both prevention of the disorder and treatment of the disorder in a clinically symptomatic individual.

By the terms "effective amount" or "pharmaceutically effective amount" or "an effective anti-estrogenic amount" of an agent as provided herein are meant a nontoxic but sufficient amount of the agent to provide the desired prophylactic or therapeutic effect. As will be pointed out below, the exact amount required will vary from subject to subject, depending on the age and general condition of the subject, the severity of the condition being treated, and the particular phytoestrogen employed and mode of administration Thus, it is not possible to specify an exact "effective amount". However, an appropriate "effective" amount in any individual case may be determined by one of ordinary skill in the art using only routine experimentation.

By "pharmaceutically acceptable carrier" is meant a material which is not biologically or otherwise undesirable, i.e., the material may be administered to an individual along with the selected phytoestrogen without causing any undesirable biological effects or interacting in a deleterious manner with any of the other components of the pharmaceutical composition in which it is contained.

Cancer is the growth of new cells in the body wherein the new cells typically have adverse effects in the body. Cancer is characterized by an increase in the number of abnormal, or neoplastic, cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites via a process called metastasis. In a cancerous state, a cell proliferates under conditions in which normal cells

would not grow. Cancer manifests itself in a wide variety of forms, characterized by different degrees of invasiveness and aggressiveness.

Administration of a phytoestrogen according to claim 1 is effective to provide anti-prostate cancer activity. It is believed that the general anti-cancer activity of wogonin, isoliquiritigenin, coumestrol is related to their activity as COX-2 inhibitors. COX-2 is a key inducible enzyme in the conversion of arachidonic acid to prostaglandins and other eicosanoids. COX-2 expression can be induced by a variety of factors; including, for example, growth factors, interleukin-1, and tumor promoting factors. The enzyme is expressed in a number of tumor cells, and human cancers, among which is prostate cancer. COX-2 inhibitors are known have use as anti-cancer therapeutics.

In addition to general anti cancer activity, the phytoestrogens wogonin, isoliquiritigenin, and coumestrol are disclosed herein as being useful as anti-hormone-related cancer agents. Hormone-related cancers include, for example, bladder cancer, bone cancer, breast cancer, colon cancer, endometrial cancer, lung cancer, ovarian cancer, prostate cancer, testicular cancer, and thyroid cancer.

In the treatment of prostate cancer, the phytoestogen composition according to claim 9 may be administered orally, parenterally, transdermally, rectally, nasally, buccally, vaginally or via an implanted reservoir in dosage formulations containing conventional non-toxic pharmaceutically acceptable carriers, adjuvants and vehicles. The term "parenteral" as used herein is intended to include subcutaneous, intravenous, and intramuscular injection. The amount of active compound administered will, of course, be dependent on the subject being treated, the subject's weight, the manner of administration and the judgment of the prescribing physician.

In combination with an anti-cancer agent, the dosage of the phytoestrogen will be 0.01 mg/kg/day to 1000 mg/kg/day, preferably 0.01 mg/kg/day to 300 mg/kg/day, more preferably 1 mg/kg/day to 300 mg/kg/day, and the dosage of anti-cancer agent will be 0.1 ug/kg/day to 100 mg/kg/day, preferably 0.3 ug/kg/day to 50 mg/kg/day, more preferably 0.01 mg/kg/day to 50 mg/kg/day.

The dosage of immune stimulant will be 1 mg/kg/day to 5000 mg/kg/day, more preferably mg/kg/day to 1000 mg/kg/day

Also disclosed herein is the use of the phytoestrogens wogonin, isoliquiritigenin, and coumestrol in the treatment of other estrogen-related disorders including, for example, bone loss, bone fractures, osteoporosis, glucocorticoid induced osteoporosis, Paget's disease, abnormally increased bone turnover, periodontal disease, tooth loss, rheumatoid arthritis, osteoarthritis, periprosthetic osteolysis, osteogenesis imperfecta, metastatic bone disease, hypercalcemia of malignancy, cartilage degeneration, endometriosis, uterine fibroid disease, hot flashes, cardiovascular disease, impairment of cognitive function, cerebral degenerative disorders, restenosis, gynecomastia, vascular smooth muscle cell proliferation, obesity, incontinence, and combinations thereof. As used herein, estrogen-related disorder also includes the symptoms of menopause, the transition from the reproductive stage to the non-reproductive stage of a woman's life, characterized primarily by the cessation of menstruation. Symptoms of menopause include, for example, hot flashes, sweating secondary to vasomotor instability, psychological and emotional symptoms of fatigue, insomnia, irritability and nervousness, lack of sleep, dizziness, cardiac symptoms; the incidence of heart disease increases, nausea, constipation, diarrhea, arthralgia, myalgia, and combinations of the foregoing symptoms.

Osteoporosis, or loss of bone density, results in increased bone fractures and vertebral column collapse. Bone loss often begins around age 35. This loss accelerates during menopause, which generally occurs around age 45 to 55. Bone mass losses average about 1-2% each year after menopause. The primary sites are the vertebrae, which show anterior collapse resulting in stooping and backache, the hips and the wrist. Osteoporosis develops over decades and is related to peak bone mass, as well as to the degree of bone loss.

In the treatment of an estrogen-related disorder, the phytoestrogen compositions may be administered orally, parenterally, transdermally, rectally, nasally, buccally, vaginally or via an implanted reservoir in dosage formulations containing conventional non-toxic pharmaceutically acceptable carriers, adjuvants and vehicles. The term "parenteral" as used herein is intended to include subcutaneous, intravenous, and intramuscular injection. The amount of active compound administered will, of course, be dependent on the subject being treated, the subject's weight, the manner of administration and the judgment of the prescribing physician. Generally, however, the dosage of phytoestrogen will be about 0.1 mg/kg/day to about 1000 mg/kg/day, more preferably about 0.1 mg/kg/day to about 300 mg/kg/day.

The invention is further illustrated by the following examples.

## GENERAL EXPERIMENTAL

Wogonin was extracted from 7 grams of a multi-component botanical extract composition containing extracts of Panax pseudo-ginseng Wall, Isatis Indigotica Fort, Ganoderma lucidium Karst, Dendrathema morifolium Tzvel, Glycyrrhiza glabra L, Sculletaria bailcalensis Georgi, Rabdosia rubescens, and Serenoa repens. The powder was dissolved in 150 milliliters of acetone using a soxhlet extractor for one hour. The liquid phase extract was further purified by silica gel column chromatography using a solvent system of 2:1 cyclohexane:acetone. About 25 milligrams of the yellow powder was obtained from fractions 11-13 (8 milliliters per fraction). The powder was further re-crystallized from absolute

ethanol to yield yellow crystals.

Figure 1 is a high performance liquid chromatogram showing the location of wogonin in the elution profile. The chromatogram was obtained with a Shimadzu SPD-M10A chromatograph using a C18 reverse phase column and two solvent systems of water and acetonitrile in 0.1% trifluoracetic acid.

The chemical structure and molecular weight of the yellow crystal was determined by total (Fig. 2(b)) and DEPT (Fig. 2(a)) $^{13}C$ NMR (d-4 methanol solvent analyzed on Varian$^{UNITY}$ Inova 400 system). The sample was also analyzed by electronionization with a Hewlet Packard VG 7070; the mass spectrum is shown in Figure 3 and indicates a molecular weight of 284, consistent with wogonin.

Isoliquiritigenin was purified from the flavonoid fraction of *Glycyrrhiza glabra* concentrated powder (purchased from Shanghai Zhao Wei Technology Development Co.) extracted by absolute ethanol. About 1 gram of the flavonoid concentrate (dissolved in 5 milliliters of water) was passed through Sephadex LH-20 column (2.5x30 millimeters) and eluted by a gradient of methanol-water mixed solvent. Crude isoliquiritigenin was obtained at fraction 27 (10 milliliters per fraction). The crude product was further purified by silica gel chromatography eluted by mixed solvent of methylene chloride: methanol (5:1).

The chemical structure and the molecular weight of isoliquiritigenin were determined by the absorption spectrum (Fig. 5) associated with an HPLC separation (Fig. 4) performed on a Shimadzu SPD-M10A chromatograph, as well as the $^{13}C$ NMR spectra (Fig. 6(a), DEPT; Fig. 6(b), total) and mass spectrum (Fig. 7). The absorption spectrum was identical to that of the reference compound isoliquiritigenin purchased from Sigma Chemical Co, (St. Louis, Missouri).

The anti-cancer activities of wogonin and isoliquiritigenin were evaluated by determining their abilities to inhibit cancer cell growth, to modulate the cancer cell cycle, and to activate estrogen receptors.

Cancer cell lines: LNCaP, DU-145, and MCF-7 cells were purchased from the American Type Culture Collection. PTX 10, a taxol-resistant ovarian cancer cell line, was obtained from the Brander Cancer Research Laboratory, New York Medical College. Cells were maintained in RPMI 1640 culture media supplemented with 10% heat-inactivated FBS, 5 millimolar glutamine, 50 units/milliliter of penicillin G, and 50 grams/milliliter of streptomycin. The cells were routinely seeded at 1X 105 cells/milliliter in T-75 flasks, allowed to attach overnight, then treated with the herbal extract. At different times, cells were harvested by trypsinization.

EXAMPLE 1

This example demonstrates the activity of wogonin and isoliquiritigenin in inhibiting the growth of the hormone-sensitive prostate cancer cell line, LNCaP.

The MTT assay (MTT = 3-(4,5-dimethylthiazol-2-yl)-2,5-diphenyl tetrazolium bromide) was used to count viable cells. The assay reagents were purchased from Boehringer Mannheim (Roche Diagnosis Corp, Indianapolis, Indiana). In this assay, the tetrazolium dye MTT is cleaved to form formazan by metabolically active cells and exhibits a strong red absorption band at 550-618 nm. The protocol for the cell , viability assay was provided by the manufacturer and modified in our laboratory as described below.

Prostate or breast (reference) cancer cells were seeded in 96 well microtiter plates at a concentration of $3x10^3$ cells per well (MCF-7; breast cancer cells) or $6x10^3$ cells per well (LNCaP or DU145; prostate cancer cells), in a volume of 100 microliters of cell culture medium. After 24 hours, 20 microliter aliquots of the compounds at various concentrations were added to the attached cells. Each concentration was repeated in 3 different wells to obtain mean values. To eliminate any solvent effect, 20 microliters of the solvent used in the preparation of the highest concentration of the compounds (a maximum of 0.5 % by volume of dimethylsulfoxide (DMSO)) was added to the control cells in each well. The plates were incubated at 37°C in a $CO_2$ incubator for 72 hours. At the end of day 3 (after 72 hours), the culture medium was carefully removed without disturbing the cells and replaced by 100 microliters of fresh cell medium. Ten microliters of MTT reagent was added to each well and the plates were incubated again in a $CO_2$ incubator at 37°C for 4 hours. One hundred microliters of sodium dodecylsulfate (SDS) solubilizing reagent (from Boehringer Mannheim) was added to each well. The plate was allowed to stand overnight in the $CO_2$ incubator and read by an ELISA Reader (EL800, Bio-Tek Instruments, Inc.) at a wavelength of 570 nm. The percent cell viability was calculated according to the equation below:

$$V = 100\left(\frac{A_{control} - A_{treated}}{A_{control}}\right)$$

where $V$ is the percent cell viability, $A_{control}$ is the absorption of the control cells, and $A_{treated}$ is the absorption of the treated cells.

Figures 8 and 9 demonstrate the activity of wogonin and isoliquiritigenin respectively, in inhibiting the growth of LNCaP (androgen-dependent) and DU-145 (androgen-independent) prostate cancer cells. It is apparent that the inhibition of cell growth is dose dependent. The concentration of the compounds resulting in 50% inhibition of cancer cell growth, defined as $ED_{50}$, was determined by linear interpolation. $ED_{50}$ values for the two compounds obtained from these measurements are shown in Columns 1 and 2 of Table 1.

Table 1. $ED_{50}$ values from MTT Assay as a Function of Compound and Cell Type

| | LNCaP (μg/ml) | DU145 (μg/ml) | MCF7 (μg/ml) |
|---|---|---|---|
| Wogonin | 10.00 | 18.6 | NA |
| isoliquiritigenin | 3.51 | 7.60 | 3.25 |

EXAMPLE 2 (reference example)

This example demonstrates the activity of isoliquiritigenin in inhibiting the growth of the breast cancer cell line, MCF-7.

The same protocols described in Example 1 were used to evaluate the effects of isoliquiritigenin on MCF-7 cells. MCF-7 is a breast cancer cell line that expresses estrogen receptors. Therefore it is a good model to study the effect of the anti-cancer agents on estrogen-receptor positive breast cancer.

Figure 9 shows the MTT assay curves for isoliquiritigenin with MCF-7. The data show that isoliquiritigenin inhibited the growth of MCF-7 cells, and dosage-dependent curves were observed. $ED_{50}$ values are given in Column 3 of Table 1, above.

EXAMPLE 3

This example demonstrates modulation of the LNCaP cell cycle by wogonin and isoliquiritigenin. LNCaP cells have a hormone-dependent cell cycle.

Sample preparation for cell cycle measurement: Cultured cells (2-4 x $10^6$ cells) were exposed to two concentrations each of wogonin and isoliquiritigenin for 24-48 hours in 12.5 $cm^2$ area flasks before being harvested. The cells were washed with phosphate buffered saline (PBS) and fixed in ice-cold 70% ethanol. Aliquots of fixed cells were rehydrated in PBS and stained with 1.0 microgram/milliliter DAPI (4,6-diamidino-2-phenylindole from Eastman Kodak, Rochester, NY), and dissolved in 10 millimolar piperazine-N,N-bis-2-ethane-sulfonic acid buffer (Calbiochem, La Jolla, CA) containing 100 millimolar NaCl, 2mM $MgCl_2$ and 0.1% Triton X-100 (Sigma) at pH 6.8 as previously described by Halicka et al. (H. D. Halicka, B. Ardelt, G. Juan, A. Mittelman, S. Chen, F. Traganos and Z. Darzynkiewicz, "Apoptosis and Cell Cycle Effects Induced by Extracts of the Chinese Herbal Preparation of PC SPES", *International J. of Oncology* (1997), 11: 437-448).

The cellular DNA content was measured with an ELITE ESP flow cytometer (Coulter Inc., F1.) using UV laser illumination. The multicycle program was used to deconvolute the DNA frequency histograms to estimate the frequency of cells in different phases of the cell cycle.

Figure 10 displays the DNA histograms of LNCaP cells in the presence and absence of wogonin at 3 micrograms/milliliter after 24 hours. It is evident that there was a change at the G1 phase as shown by the arrow bar. Data analysis revealed the increase in G1 phase was proportional to wogonin concentration.

Similar measurements were conducted for isoliquiritigenin. Figure 11 summarizes the effects of wogonin and isoliquiritigenin on G1, S, and $G_2M$ phases of the LNCaP cell cycle. The data show that wogonin induced a G1 phase arrest, and isoliquiritigenin induced a $G_2M$ phase arrest. A prolongation in either G1 or $G_2M$ phases leads to the suppression of LNCaP cell proliferation.

EXAMPLE 4

This example demonstrates modulation of the DU-145 cell cycle by wogonin and isoliquiritigenin.

The protocol described in Example 3 was used to study the effect of wogonin on the hormone-independent prostate cancer cell line DU-145. The results are presented in Figure 12 and show that wogonin prolonged the $G_2M$ phase of DU-145. A prolongation in the $G_2M$ phase leads to the suppression of DU-145 cell proliferation.

EXAMPLE 5 (reference)

The estrogenic activity of wogonin and isoliquiritigenin were demonstrated by determining their ability to activate estrogen receptors (subclass alpha and beta).

HEK 293 cells (ATCC CRL-1573) were transfected with an expression vector for $hER_{\alpha}$ and $hER_{\beta}$ respectively, and an ERE-LUC reporter gene (plus a TK-LUC reporter for normalization) following the protocol of Yoon et al ("Differential activation of wild-type and variant forms of estrogen receptor $\alpha$ by synthetic and natural estrogenic compounds using a promoter containing three estrogen-responsive elements", *J. Steroid Biochem. & Molecular Biology* (2000), 28: pages 25-32).

Cells were then separately exposed to wogonin or isoliquiritigenin at concentrations of 0, 0.07, 0.02, 0.08, 0.3, 0.7, 1.4 (or 4) and 9 μg/ml for 20 hours and then cell lysates were assayed for reporter gene expression (Tzukerman et al. "Human estrogen receptor transactivational capacity is determined by both cellular and promoter content and mediated by two functionally distinct intermolecular regions", *Mol. Endocrinol.* (1994), 8: 21-30).

Figure 13 shows the dose-responsive behavior of ERα-Luc reporter gene activated by wogonin and isoliquiritigenin.

Figure 14 shows the dose-responsive behavior of ERβ-Luc reporter gene activated by wogonin and isoliquiritigenin. It is very significant that wogonin and isoliquiritigenin showed at least 10 times more capability in activating ERβ-Luc reporter gene than the ERα-Luc reporter gene.

EXAMPLE 6

This example demonstrates the inhibition of COX-2 activity by isoliquiritigenin.

COX is a bifunctional enzyme that exhibits both cyclooxygenase and peroxidase activities. The cyclooxygenase activity is responsible for the oxidation of arachidonic acid to Prostaglandin $G_2$ ($PGG_2$) and the peroxidase activity is responsible for the subsequent reduction of $PGG_2$ to the corresponding alcohol, $PGH_2$. Some methods used to determine COX inhibitor activity include measuring uptake of oxygen using an oxygraph, measuring the conversion of radioactive arachidonic acid, or measuring the prostaglandins formed from $PGH_2$ (using immunoassay techniques).

These experiments employed the ovine COX-2 Inhibitor Screening Assay commercially available as catalog no. 760101 from Cayman Chemical (Ann Arbor, MI 48108). This immunoassay uses an antibody to that binds all major prostaglandins to measure quantitatively the amount of $PGF_{2\alpha}$ produced in the COX reaction using arachidonic acid as a substrate. The final volume of the reactions as described below is typically 1.15 ml. In brief, COX-1 or COX-2 (e.g., 20 μl of a solution of from 1 to 100 units/ml COX, where a unit is defined as the amount of enzyme that consumes one nanomole of oxygen per minute at 37°C in 0.1 M Tris-HCl buffer, pH 8.0 containing 100 μM arachidonate, 5 mM EDTA, 2 mM phenol and 1 μM hematin) is first mixed with a buffer (e.g., 0.1 M Tris, pH 8.0, 5 mM EDTA, 2 mM phenol) and heme (e.g., 10 μl of 10 mM heme) in a microfuge tube. The heme is a cofactor for COX that provides maximal activity in the assay. COX samples that contain an inhibitor can be pre-mixed with the inhibitor (e.g., 1-100 μM) prior to adding substrate. The arachidonic acid substrate (e.g., 10 μl of a 10 mM solution) can be then added to the COX/heme/inhibitor mixture for a time and at a temperature sufficient for the reaction to proceed to produce a detectable product (e.g., 2 minutes at 37°C). The reaction can be quenched with acid (e.g., 50 μl of 1 M HCl). Additionally stannous chloride (e.g., 100 μl of a saturated solution) can be added to convert the $PGH_2$ produced to the more stable $PGF_{2\alpha}$ for the purpose of quantification of prostaglandin. The prostaglandin produced in the reactions is typically quantified using an enzyme immunoassay.

The enzyme immunoassay to detect prostaglandin can conveniently be performed in a 96 well plate using an antibody to detect prostaglandin. As controls, prostaglandin standards and COX 100% activity samples (no inhibitor) can be measured. Samples used for background correction can also be used. Controls and reactions performed with COX and the inhibitors of interest are incubated with prostaglandin screening antiserum in an amount sufficient to detect the prostaglandin produced in the COX reactions (e.g., 50 μl of antiserum diluted in 6 ml of a suitable EIA buffer). The reactions can be incubated for a time and temperature sufficient to allow interaction of the antiserum and the prostaglandins (e.g., 18 hours at room temperature). When ready to develop the plate, the reactions are first washed and then incubated with 200 μl of Ellman's reagent for 60 minutes or so. The plate can be read at 405 to 420 nm on a plate reader. The prostaglandin standards are used to calculate a standard curve of prostaglandin concentration. The amount of prostaglandin in each sample with inhibitor is subtracted from the amount of prostaglandin in the 100% activity sample, divided by the amount of prostaglandin in the 100% activity sample, and multiplied by 100 to give the percent inhibition. Graphing the percent inhibition vs. the inhibitor calculation allows the calculation of the $IC_{50}$ value (the concentration at which there is 50% inhibition).

Methods of measuring the activity of a COX inhibitor are described in, for example, W. Xie, J. G. Chipman, D. L. Robertson, et al., "Expression of a mitogen-responsive gene encoding prostaglandin synthase is regulated by mRNA splicing", *Proc. Natl. Acad. Sci.USA* (1991), 88: 2692-2696; K.M. Maxey, K.R. Maddipati, and J. Birkmeier, "Interference

in enzyme immunoassays", *J. Clin. Immunoassay* (1992), 15: 116-120; P. Pradelles, J. Grassi, and J. A. Maclouf, "Enzyme immunoassays of eicosanoids using acetylcholinesterase as label: An alternative to radioimmunoassay", *Anal. Chem.* (1985), 57: 1170-1173; Maclouf, J., Grassi, J., and Pradelles, P. Development of enzyme-immunoassay techniques for the measurement of eicosanoids, *Prostaglandin and Lipid Metabolism in Radiation Injury* (1987), pages 355-364.

Figure 15 displays the dose-dependent inhibitory activity of isoliquiritigenin on COX-2 measured according to the procedure above. An $IC_{50}$ of 10.5 $\mu$M was calculated from the data.

EXAMPLE 7: demonstrates the cytotoxicity of wogonin and isoliquiritigenin on an ovarian cancer cell line (reference example)

PTX 10, a taxol resistant ovarian cancer cell line, cells were maintained in RPMI 1640 medium supplemented with 10% fetal calf serum, 100 units/ml penicillin, 100 mg/ml streptomycin, and 2mM L-glutamine (all from Gibco/BRL Life Technologies, Inc., Grand Island, NY) at 37.5°C in an atmosphere of 5% $CO_2$ in air. At the onset of experiments the cultures were at the densities below $5\times10^5$ cells/ml and the cells were growing exponentially and asynchronously.

The MTT assay was performed to study the effect of wogonin and isoliquiritigenin on the cell growth of PTX 10. The MTT assay protocol is the same as that described in Example 1. Figures 16 and 17 display the inhibition curves of PTX cell line in the presence of wogonin and isoliquiritigenin respectively. A concentration- dependent inhibition was clearly observed in these two figures. The $ED_{50}$ calculated from the inhibition curves are 1.56 ug/mL and 3.32 ug/mL for wogonin and isoliquiritigenin respectively as shown in Table 2.

Table 2 $ED_{50}$

| | PTX 10 (taxol resistant Ovarian cancer cell) |
|---|---|
| Wogonin (I -16-2) | < 1.56 ug/mL |
| Isoliquiritigenin | 3.32 ug/ml |

Thus, wogonin and isoliquiritigenin may have utility for treating cancers that are resistant to treatment by other agents such as, for example, taxol.

EXAMPLE 8

A composition according to this disclosure was administered in capsules 6 times a day to two elderly volunteer patients diagnosed with prostate cancer. As a measure of the progress of the cancer, the bloodstream level of prostate-specific antigen (PSA), a substance produced by the prostate gland, was measured by standard methods. The results are shown in Table 3.

Table 3

| Patient Age | Treatment prior to treatment with Composition 1 | PSA at start of treatment, ng/mL | PSA after 1 month of treatment, ng/mL | PSA after 2 months of treatment, ng/mL | Percent PSA reduction after 1 month of treatment | Percent PSA reduction after 2 months of treatment |
|---|---|---|---|---|---|---|
| 73 | Castration plus Flutamide | 80 | 40 | 6 | 50% | 92.5% |
| 79 | Prostatectomy; no medication | 120 | 64 | 18 | 46.7% | 85% |

As can be seen from table 1, a dramatic reduction in PSA levels is observed after 1 and 2 months of treatment with composition 1.

Compositions comprising wogonin, isoliquiritigenin, coumestrol and combinations thereof have both phytoestrogenic and anti-cancer activities. The claimed compositions comprise an additional anti-cancer agents and immune stimulant, and/or immune stimulants. The anti-cancer activity is demonstrated by the ability of wogonin and isoliquiritigenin to inhibit the growth of cancer cell lines. The identification of isoliquiritigenin as a COX-2 inhibitor suggests that it has general anti-cancer activity. The identification of the phytoestrogenic activity of wogonin suggests that, in addition to general anti-cancer activity due to COX-2 inhibition, wogonin may be particularly useful in the treatment of hormone-related cancers.

## Claims

1. The use of a phytoestrogen in the manufacture of a medicament for treating prostate cancer, wherein said phytoestrogen comprises greater than 0.5 weight percent of the medicament based on the total weight of the medicament and said phytoestrogen is selected from the group consisting of isoliquiritigenin, its pharmaceutically acceptable salts and glycoside esters, and a combination of one or more of the foregoing compounds; and
wherein the medicament further comprises an anti-cancer agent selected from the group consisting of oridonin, indirubin, taxol, cis-platin, camptothecan, vincristine, monocrotaline, Maytansine, homoharringtonine, colchicine, irisquinone A, irisquinone B, irisquinone C, acronycine, matrin, oxymatrin, curcumin, paricine, pariphyllin, and combinations comprising one or more of the foregoing anti-cancer agents; and
an immune stimulant selected from the group consisting of ginsenosides, ferulic acid, mannan, synanthrin, eleutheroside A, eleutheroside B, eleutheroside C, eleutheroside D, eleutheroside E, gynoside, beta-pachyman, inulin, glycoproteins, interferons, γ-globulins, extracts of *Ganoderma lucidum,* extracts of *Coriolus versicolor,* extracts of *Poria cocos,* and combinations comprising one or more of the foregoing immune stimulants.

2. The use of claim 1 wherein said phytoestrogen comprises greater than 2 weight percent of the medicament based on the total weight of the medicament.

3. The use of claim 1 wherein said phytoestrogen comprises greater than 5 weight percent of the medicament based on the total weight of the medicament.

4. The use of claim 1 wherein said phytoestrogen comprises greater than 10 weight percent of the medicament based on the total weight of the medicament.

5. The use of claim 1 wherein said phytoestrogen comprises greater than 20 weight percent of the medicament based on the total weight of the medicament.

6. The use of any one of Claims 1 to 5, wherein the medicament comprises an extract of *Glycyrrhiza uralensis, Glycyrrhiza glabra,* or a combination of the foregoing compounds.

7. The use of any one of Claims 1 to 6, wherein the medicament further comprises wogonin, coumestrol, or a combination of the foregoing compounds.

8. The use of any one of Claims 1-7, wherein said medicament is formulated for administering a dosage of 0.01 mg/kg/day to 300 mg/kg/day of isoliquiritigenin.

9. A composition, comprising:

   greater than or equal to 0.5 weight percent based on the total weight of the composition of isoliquiritigenin, its pharmaceutically acceptable salts or glycoside esters, or combinations comprising one or more of the foregoing compounds; and
   at least one anti-cancer agent selected from the group consisting of oridonin, indirubin, taxol, cis-platin, camptothecan, vincristine, monocrotaline, Maytansine, homoharringtonine, colchicine, irisquinone A, irisquinone B, irisquinone C, acronycine, matrin, oxymatrin, curcumin, paricine, pariphyllin, and combinations comprising one or more of the foregoing anti-cancer agents; and
   an immune stimulant selected from the group consisting of ginsenosides, ferulic acid, mannan, synanthrin, eleutheroside A, eleutheroside B, eleutheroside C, eleutheroside D, eleutheroside E, gynoside, beta-pachyman, inulin, glycoproteins, interferons, γ-globulins, extracts of *Ganoderma lucidum,* extracts of *Coriolus versicolor,* extracts of *Poria cocos,* and combinations comprising one or more of the foregoing immune stimulants.

10. The composition of Claim 9, further comprising wogonin, coumestrol, or a combination of the foregoing compounds.

11. The composition of Claim 9 or 10, wherein said anti-cancer agent is oridonin and said immune stimulant is beta-pachyman.

12. A composition as claimed in any one of Claims 9 to 11 wherein said phytoestrogen comprises the weight percent of the medicament as defined in any one of claims 2 to 5.

13. A composition as claimed in any one of Claims 9 to 12 for use as a medicament.

**Patentansprüche**

1. Verwendung eines Phytoöstrogens in der Herstellung eines Medikamentes zur Behandlung von Prostatakrebs, wobei das Phytoöstrogen mehr als 0,5 Gew.-% des Medikaments, bezogen auf das Gesamtgewicht des Medikaments, umfasst und das Phytoöstrogen ausgewählt ist unter Isoliquiritigenin, seinen pharmazeutisch verträglichen Salzen und Glycosidestem und einer Kombination einer oder mehrerer der vorstehend erwähnten Verbindungen; und das Medikament außerdem ein Antikrebsmittel enthält, das ausgewählt ist unter Oridonin, Indirubin, Taxol, Cisplatin, Camptothecan, Vincristin, Monocrotalin, Maytansin, Homoharringtonin, Colchicin, Irisquinon A, Irisquinon B, Irisquinon C, Acronycin, Matrin, Oxymatrin, Curcumin, Paricin, Pariphyllin und Kombinationen eines oder mehrerer der vorstehend aufgeführten Antikrebsmittel; und
ein Immunstimulans, ausgewählt unter Ginsenosiden, Ferulasäure, Mannan, Synanthrin, Eleutherosid A, Eleutherosid B, Eleutherosid C, Eleutherosid D, Eleutherosid E, Gynosid, β-Pachyman, Inulin, Glycoproteinen, Interferonen, γ-Globulinen, Extrakten von Ganoderma lucidum, Extrakten von Coriolus versicolor, Extrakten von Poria cocos und Kombinationen, die eine oder mehrere der vorstehend aufgeführten Verbindungen umfassen.

2. Verwendung nach Anspruch 1, wobei das Phytoöstrogen mehr als 2 Gew.-% des Medikaments, bezogen auf das Gesamtgewicht des Medikaments, umfasst.

3. Verwendung nach Anspruch 1, wobei das Phytoöstrogen mehr als 5 Gew.-% des Medikaments, bezogen auf das Gesamtgewicht des Medikaments, umfasst.

4. Verwendung nach Anspruch 1, wobei das Phytoöstrogen mehr als 10 Gew.-% des Medikaments, bezogen auf das Gesamtgewicht des Medikaments, umfasst.

5. Verwendung nach Anspruch 1, wobei das Phytoöstrogen mehr als 20 Gew.-% des Medikaments, bezogen auf das Gesamtgewicht des Medikaments, umfasst.

6. Verwendung nach einem der Ansprüche 1 bis 5, wobei das Medikament einen Extrakt von Glycyrrhiza uralensis, Glycyrriza glabra oder eine Kombination der vorstehend erwähnten Verbindungen umfasst.

7. Verwendung nach einem der Ansprüche 1 bis 6, wobei das Medikament außerdem Wogonin, Cumestrol oder eine Kombination der vorstehend erwähnten Verbindungen umfasst.

8. Verwendung nach einem der Ansprüche 1 bis 7, wobei das Medikament für die Verabreichung einer Dosis von 0,01 mg/kg/Tag bis 300 mg/kg/Tag Isoliquiritigenin formuliert ist.

9. Eine Zusammensetzung, umfassend
mindestens 0,5 Gew.% an lsoliquiritigenin, seinen pharmazeutisch verträglichen Salzen oder Glycosiden oder Kombinationen einer oder mehrerer der vorstehend aufgeführten Verbindungen, bezogen auf das Gesamtgewicht der Zusammensetzung; und
mindestens ein Antikrebsmiüel, dass ausgewählt ist unter Oridonin, Indirubin, Taxol, Cisplatin, Camptothecan, Vincristin, Monocrotalin, Maytansin, Homoharringtonin, Colchicin, Irisquinon A, Irisquinon B, Irisquinon C, Acronycin, Matrin, Oxymatrin, Curcumin, Paricin, Pariphyllin und Kombinationen eines oder mehrerer der vorstehend aufgeführten Antikrebsmittel; und
ein Immunstimulans, ausgewählt unter Ginsenosiden, Ferulasäure, Mannan, Synanthrin, Eleutherosid A, Eleutherosid B, Eleutherosid C, Eleutherosid D, Eleutherosid E, Gynosid, β-Pachyman, Inulin, Glycoproteinen, Interferonen, γ-Globulinen, Extrakten von Ganoderma lucidum, Extrakten von Coriolus versicolor, Extrakten von Poria cocos und Kombinationen, die eine oder mehrere der vorstehend aufgeführten Verbindungen enthalten.

10. Zusammensetzung nach Anspruch 9, die weiterhin Wogonin, Cumestrol oder eine Kombination der vorstehend erwähnten Verbindungen umfasst.

**11.** Zusammensetzung nach Anspruch 9 oder 10, worin das Antikrebsmittel Oridon und das Immunstimulans β-Pachyman ist.

**12.** Zusammensetzung gemäß der Ansprüche 9 bis 11 mit einem Phytoöstrogenanteil am Medikament, wie er in den Ansprüchen 2 bis 5 definiert ist.

**13.** Zusammensetzung gemäß der Ansprüche 9 bis 12 zur Verwendung als Medikament.

## Revendications

**1.** Utilisation d'un phytoestrogène dans la fabrication d'un médicament de traitement du cancer de la prostate, dans lequel le phytoestrogène représente plus de 0,5 pourcent en poids du médicament par rapport au poids total du médicament et le phytoestrogène est choisi dans le groupe consistant en l'isoliquiritigénine, ses sels et esters de glycoside acceptables pharmaceutiquement, et une combinaison d'un ou de plusieurs des composés précédents ; et dans lequel le médicament comprend, en outre, un agent anticancéreux choisi dans le groupe consistant en l'oridonine, l'indirubine, le taxol, la cis-platine, le camptothécan, la vincristine, la monocrotaline, la Maytansine, l'homoharringtonine, la colchicine, l'irisquinone A, l'irisquinone B, l'irisquinone C, l'acronycine, le matrin, l'oxymatrin, le curcumin, la paricine, la pariphylline et leurs combinaisons comprenant un ou plusieurs des agents anticancéreux précédents ; et
un immunostimulant choisi dans le groupe consistant en les ginsénosides, l'acide férulique, le mannan, la synanthrine, l'éleuthéroside A, l'éleuthéroside B, l'éleuthéroside C, l'éleuthéroside D, l'éleuthéroside E, le gynoside, le bêtapachyman, l'inuline, les glycoprotéines, les interférons, les globulines γ, les extraits de *Ganoderma lucidum,* les extraits de *Coriolus versicolor,* les extraits de *Poria cocos,* et des combinaisons comprenant un ou plusieurs des immunostimulants précédents.

**2.** Utilisation suivant la revendication 1, dans laquelle le phytoestrogène représente plus de 2 pourcent en poids du médicament sur la base du poids total du médicament.

**3.** Utilisation suivant la revendication 1, dans laquelle le phytoestrogène représente plus de 5 pourcent en poids du médicament sur la base du poids total du médicament.

**4.** Utilisation suivant la revendication 1, dans laquelle le phytoestrogène représente plus de 10 pourcent en poids du médicament sur la base du poids total du médicament.

**5.** Utilisation suivant la revendication 1, dans laquelle le phytoestrogène représente plus de 20 pourcent en poids du médicament sur la base du poids total du médicament.

**6.** Utilisation suivant l'une quelconque des revendications 1 à 5, dans laquelle le médicament comprend un extrait de *Glycyrrhiza uralensis,* de *Glycyrrhiza glabra,* ou une combinaison des composés précédents.

**7.** Utilisation suivant l'une quelconque des revendications 1 à 6, dans laquelle le médicament comprend, en outre, de la wogonine, du coumestrol, ou une combinaison des composés précédents.

**8.** Utilisation suivant l'une quelconque des revendications 1 à 7, dans laquelle le médicament est formulé pour l'administration d'une posologie de 0,01 mg/kg/jour à 300 mg/kg/jour d'isoliquiritigénine.

**9.** Composition comprenant :

une quantité supérieure ou égale à 0,5 pourcent en poids par rapport au poids total de la composition d'isoliquiritigénine, de ses sels acceptables pharmaceutiquement ou de ses esters de glycoside, ou de combinaisons comprenant un ou plusieurs des composés précédents ; et
au moins un agent anticancéreux choisi dans le groupe consistant en l'oridonine, l'indirubine, le taxol, la cisplatine, le camptothécan, la vincristine, la monocrotaline, la Maytansine, l'homoharringtonine, la colchicine, l'irisquinone A, l'irisquinone B, l'irisquinone C, l'acronycine, le matrin, l'oxymatrin, le curcumin, la paricine, la pariphylline et leurs combinaisons comprenant un ou plusieurs des agents anticancéreux précédents ; et
un immunostimulant choisi dans le groupe consistant en les ginsénosides, l'acide férulique, le mannan, le synanthrin, l'éleuthéroside A, l'éleuthéroside B, l'éleuthéroside C, l'éleuthéroside D, l'éleuthéroside E, le gynoside, le bêta-pachyman, l'inuline, les glycoprotéines, les interférons, les globulines y, les extraits de *Ganoderma lucidum,* les extraits de *Coriolus versicolor,* les extraits de *Poria cocos,* et des combinaisons comprenant un ou plusieurs des immunostimulants précédents.

**10.** Composition suivant la revendication 9, comprenant en outre de la wogonine, du coumestrol, ou une combinaison des composés précédents.

**11.** Composition suivant la revendication 9 ou 10, dans laquelle l'agent anticancéreux est de l'oridonine et l'immunostimulant est du bêta-pachyman.

**12.** Composition suivant l'une quelconque des revendications 9 à 11, dans laquelle le phytoestrogène représente le pourcentage en poids du médicament tel que défini dans l'une quelconque des revendications 2 à 5.

**13.** Composition suivant l'une quelconque des revendications 9 à 12 à utiliser comme médicament.

FIG. 1

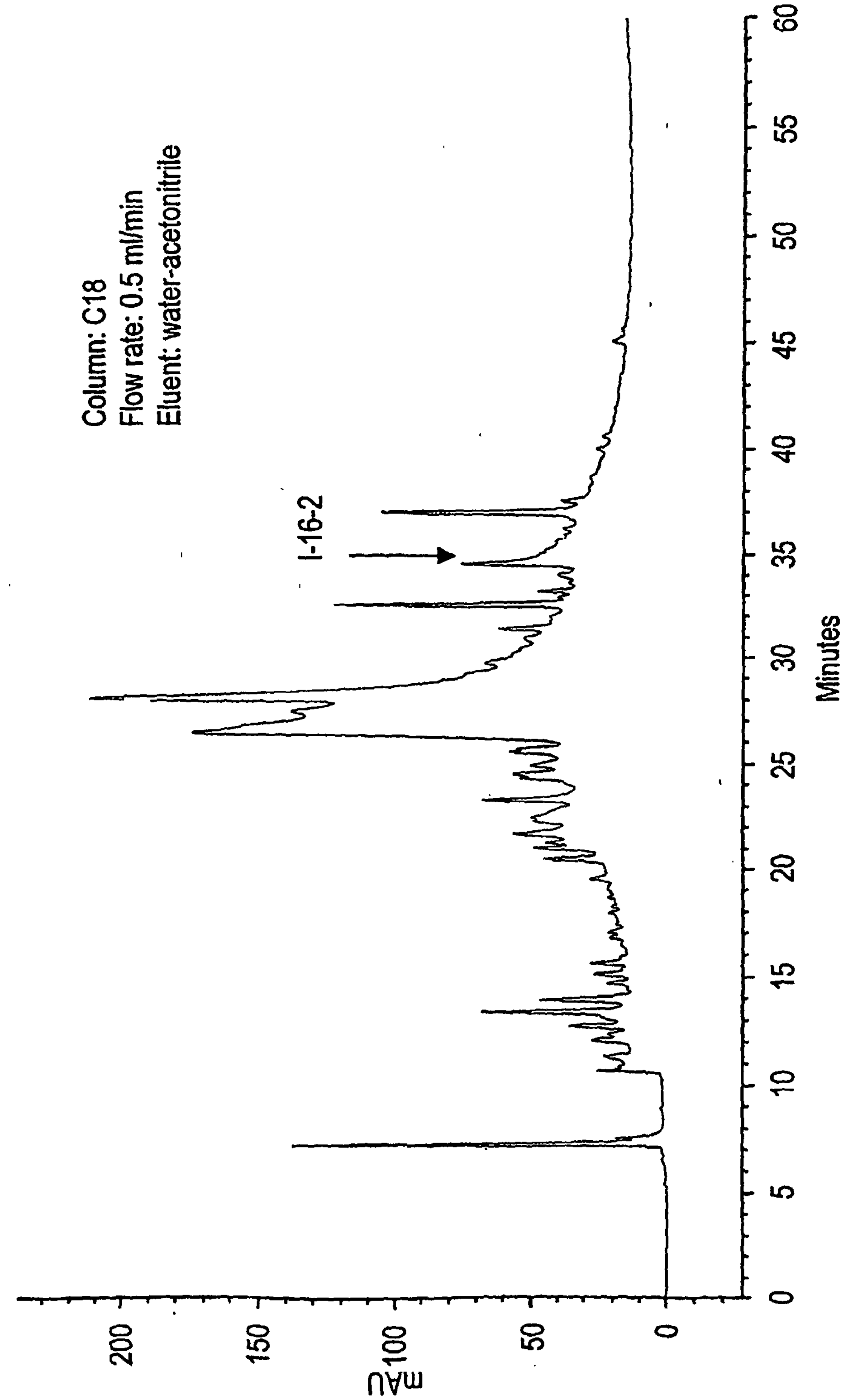
Column: C18
Flow rate: 0.5 ml/min
Eluent: water-acetonitrile
I-16-2
mAU
0
50
100
150
200
Minutes
0
5
10
15
20
25
30
35
40
45
50
55
60

FIG. 2A

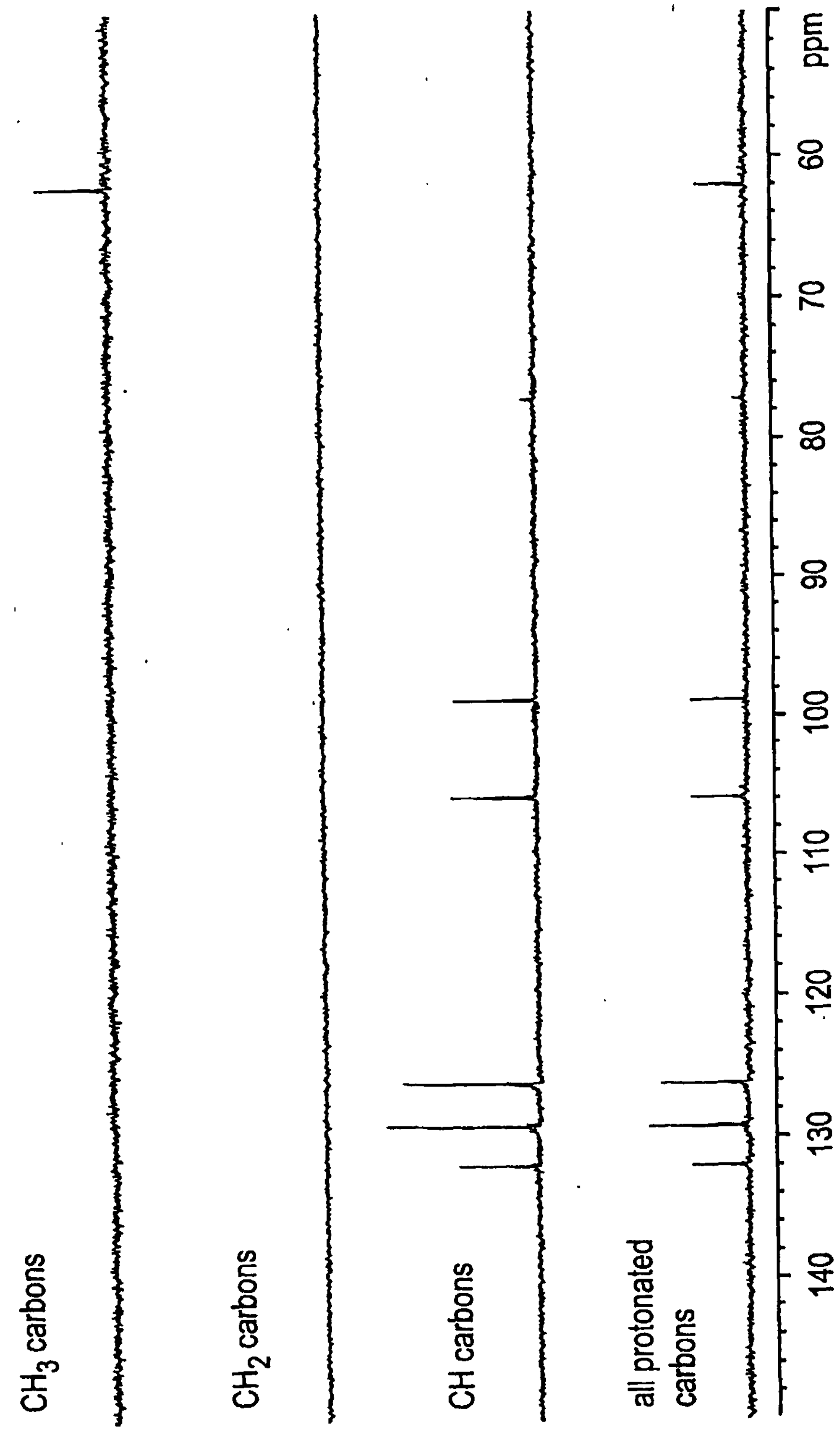
CH3 carbons
CH2 carbons
CH carbons
all protonated carbons
ppm
60
70
80
90
100
110
120
130
140

FIG. 2B

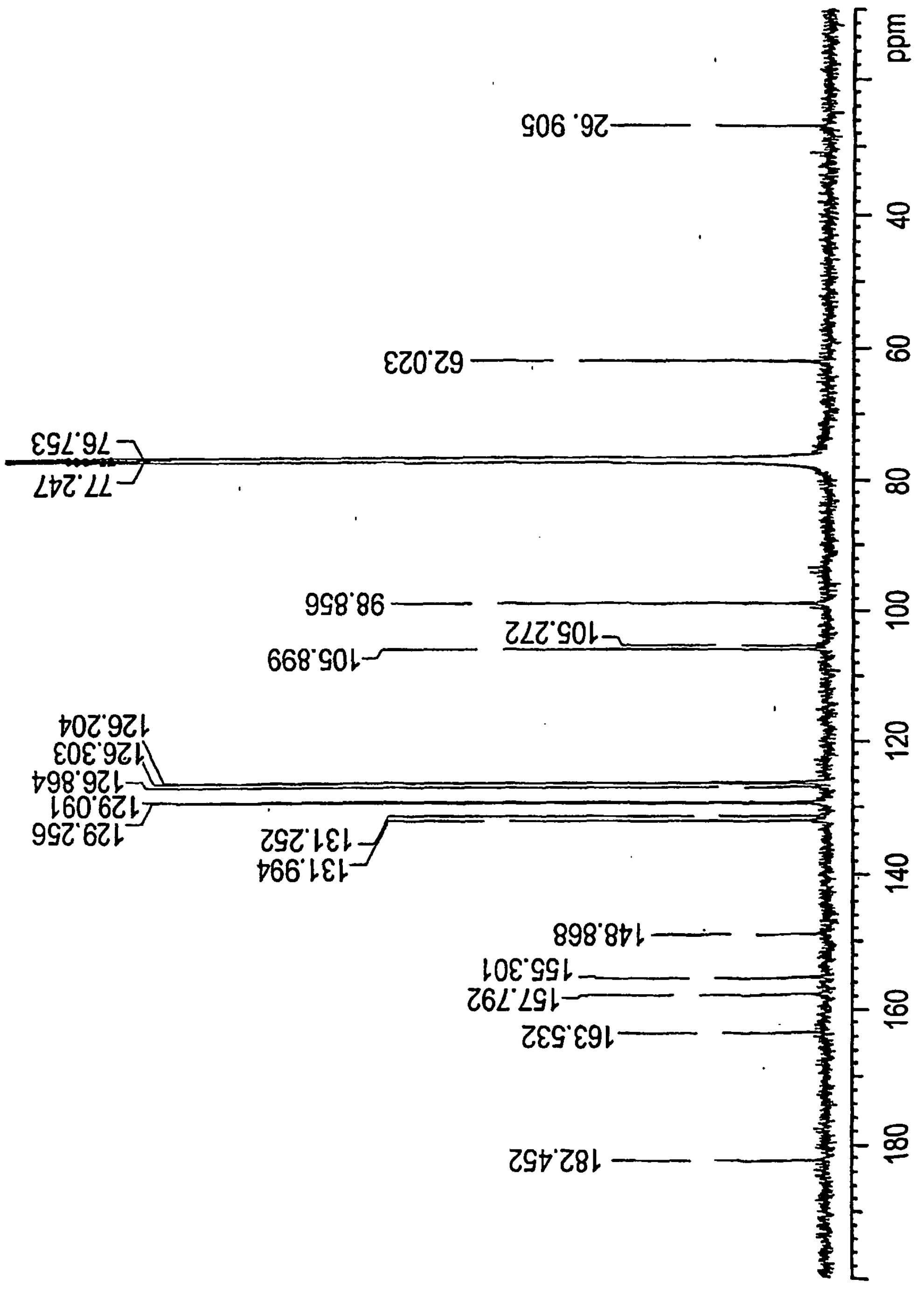
26.905
62.023
76.753
77.247
98.856
105.272
105.899
126.204
126.303
126.864
129.091
129.256
131.252
131.994
148.868
155.301
157.792
163.532
182.452
ppm
40
60
80
100
120
140
160
180

FIG. 3

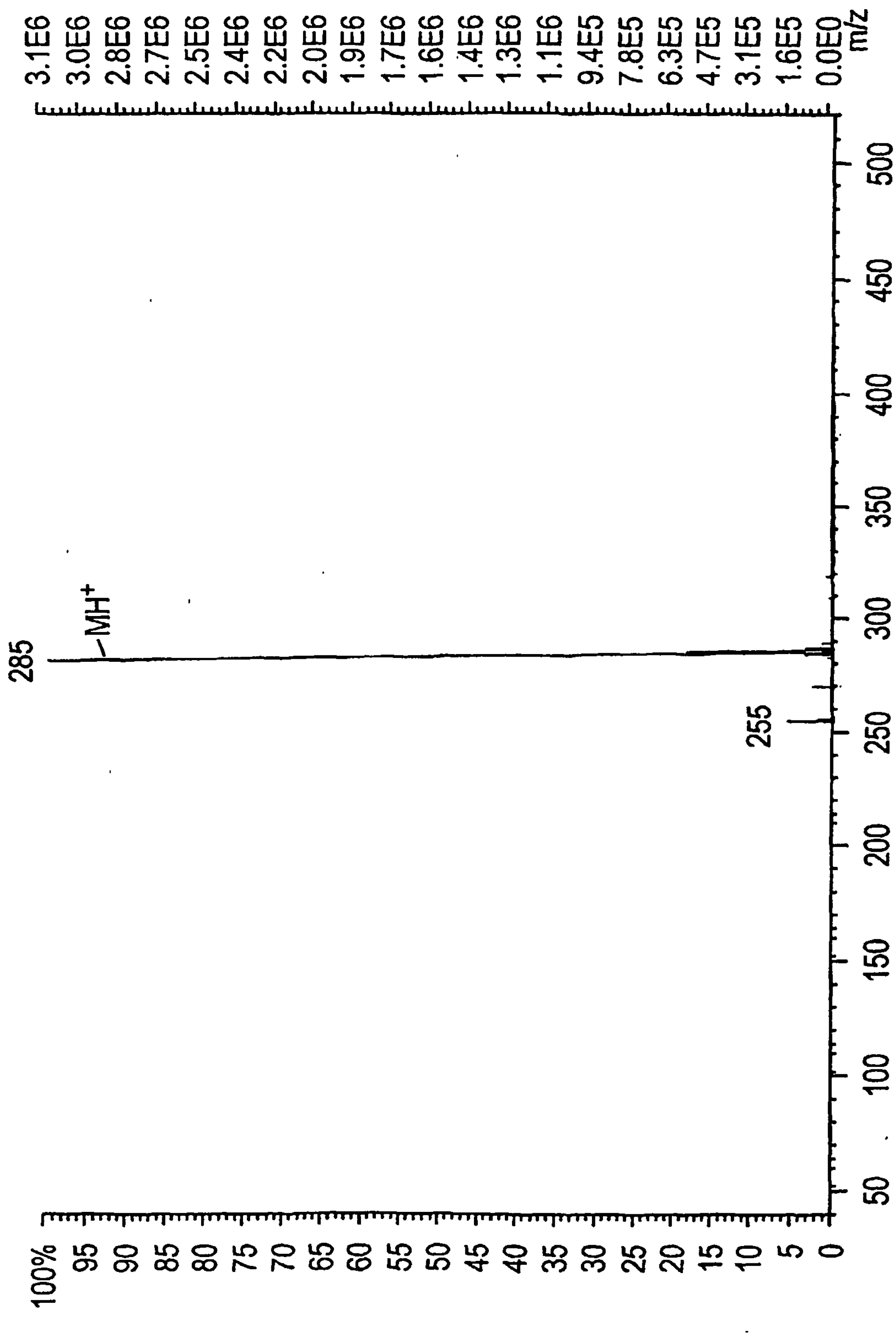

285
MH+
255
m/z
100%
95
90
85
80
75
70
65
60
55
50
45
40
35
30
25
20
15
10
5
0
50
100
150
200
250
300
350
400
450
500
3.1E6
3.0E6
2.8E6
2.7E6
2.5E6
2.4E6
2.2E6
2.0E6
1.9E6
1.7E6
1.6E6
1.4E6
1.3E6
1.1E6
9.4E5
7.8E5
6.3E5
4.7E5
3.1E5
1.6E5
0.0E0

FIG. 4

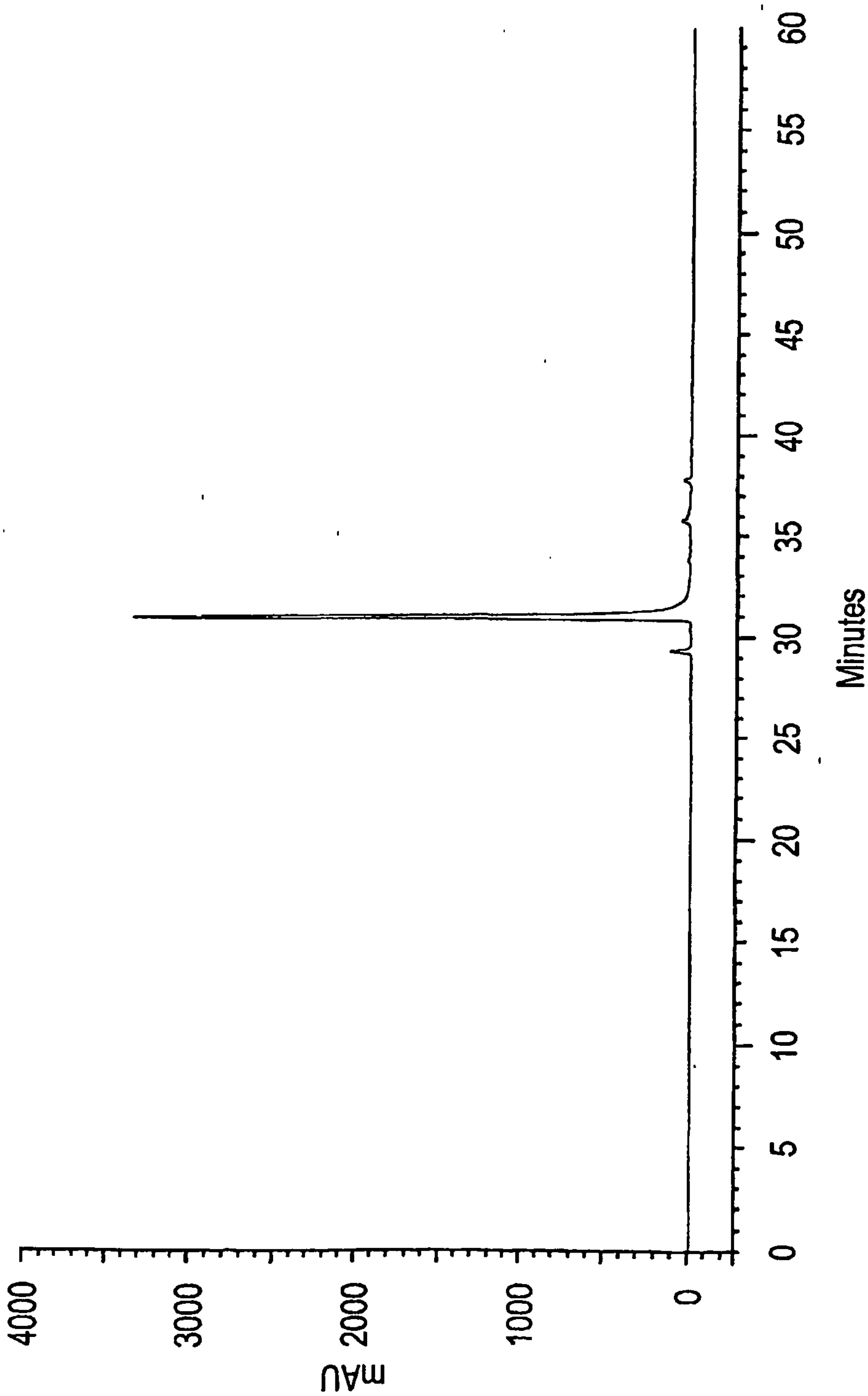

mAU
4000
3000
2000
1000
0
Minutes
0
5
10
15
20
25
30
35
40
45
50
55
60

FIG. 5

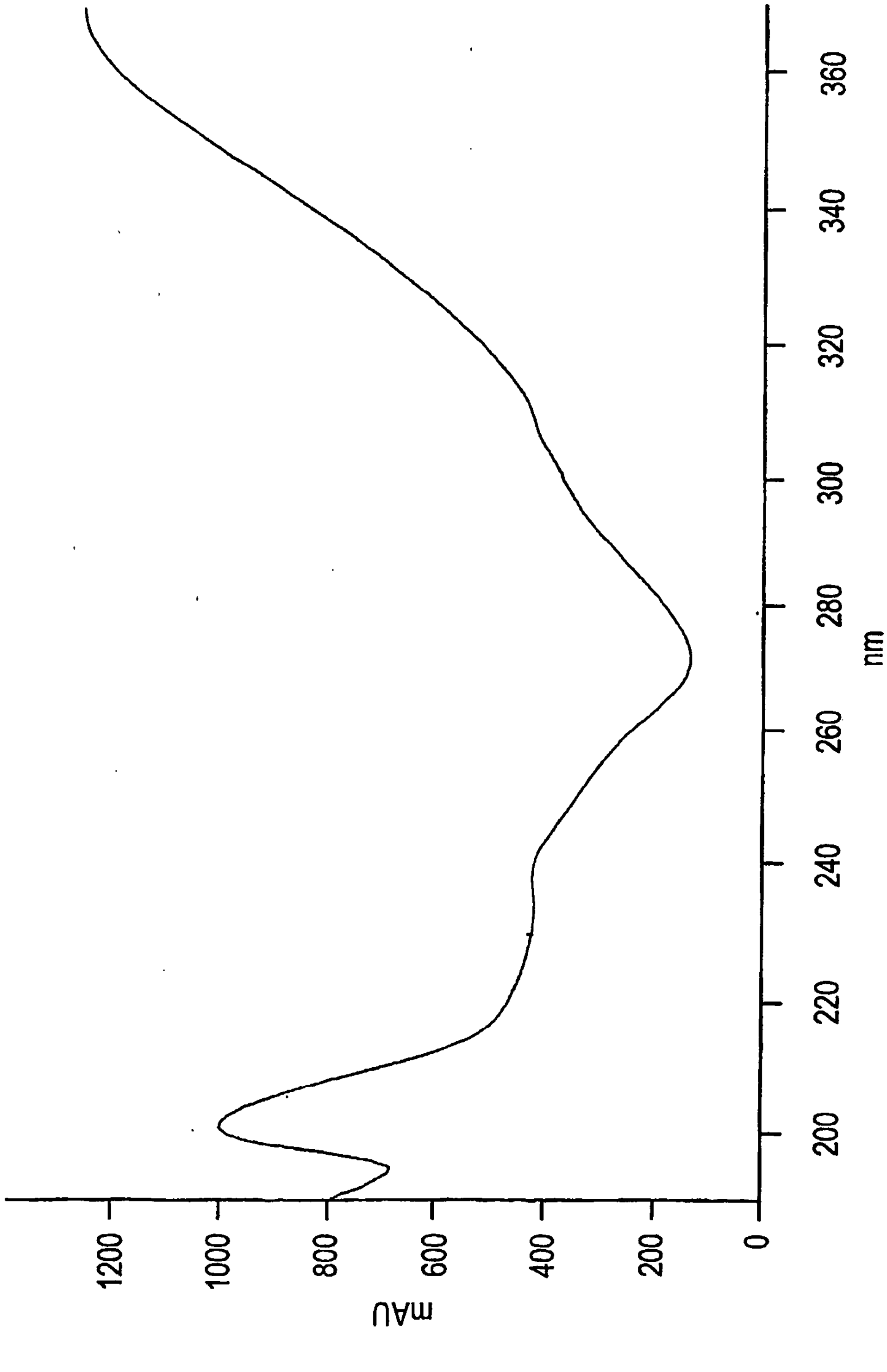
mAU
1200
1000
800
600
400
200
0
200
220
240
260
280
300
320
340
360
nm

FIG. 6A

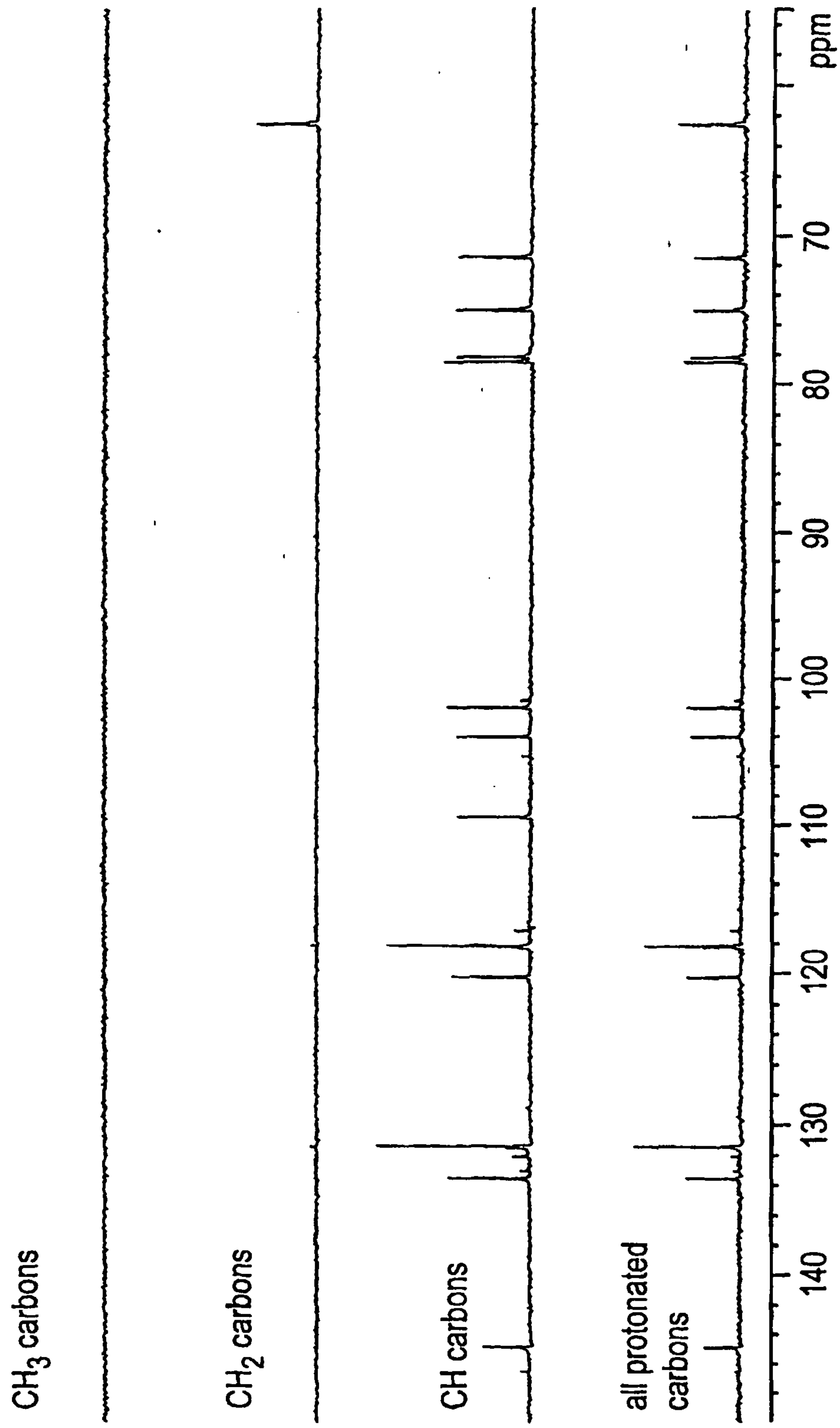
CH3 carbons
CH2 carbons
CH carbons
all protonated carbons
140
130
120
110
100
90
80
70
ppm

FIG. 6B

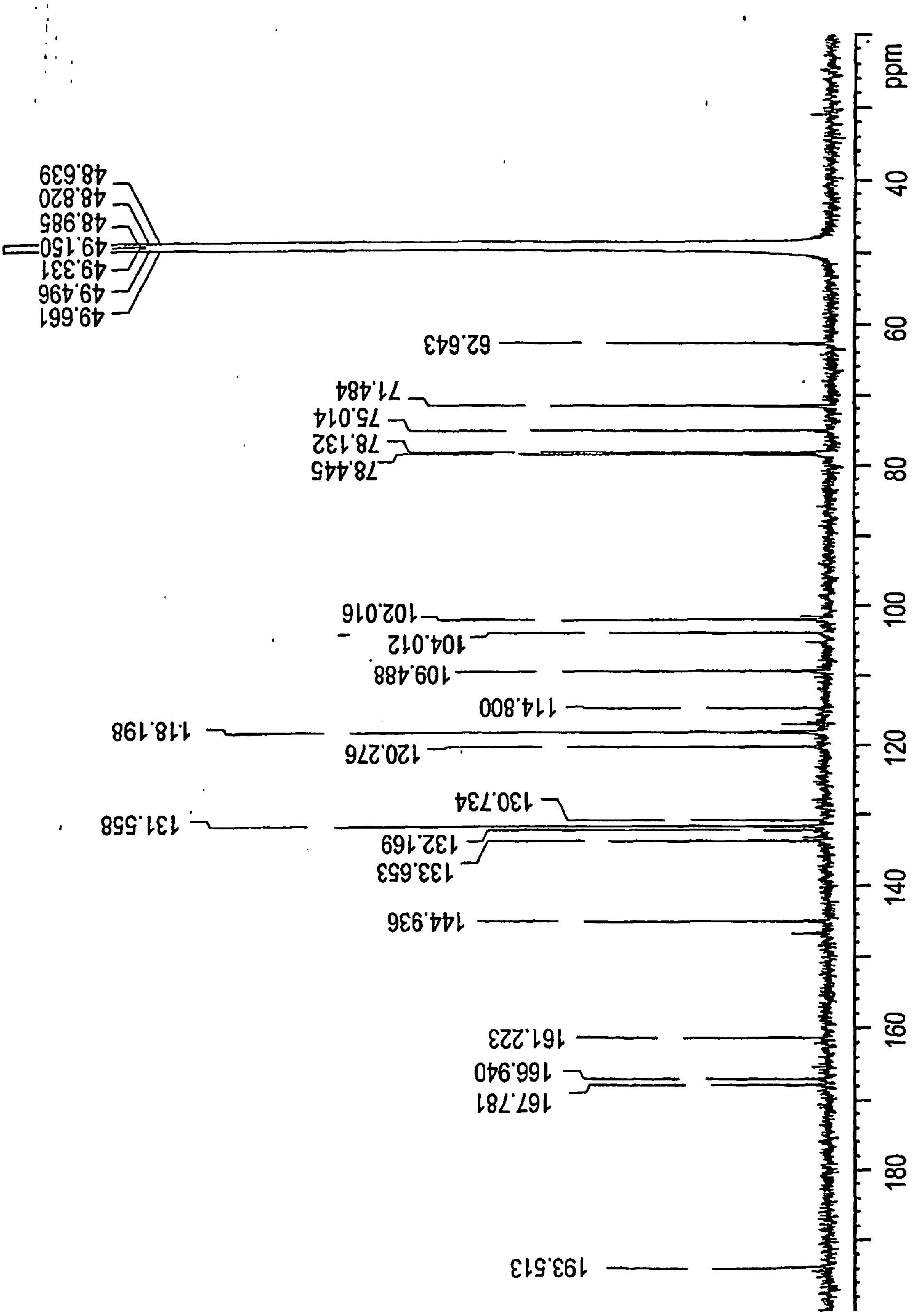
48.639
48.820
48.985
49.150
49.331
49.496
49.661
62.643
71.484
75.014
78.132
78.445
102.016
104.012
109.488
114.800
118.198
120.276
130.734
131.558
132.169
133.653
144.936
161.223
166.940
167.781
193.513
ppm
40
60
80
100
120
140
160
180

FIG. 7

Isoliquirtigenin

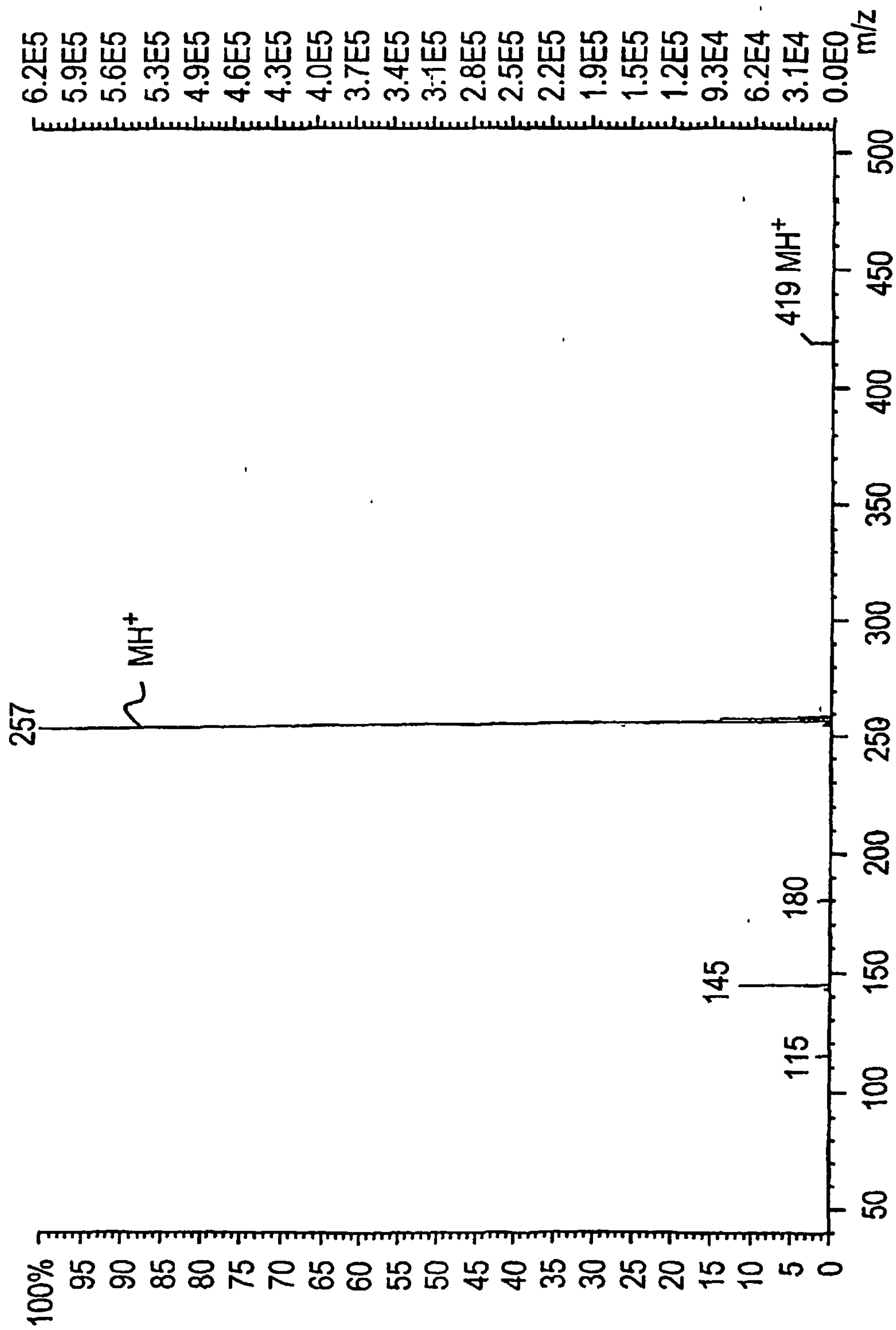

FIG. 8

Cell viability (MTT) curves for Wogonin treatment of LNCaPand DU145

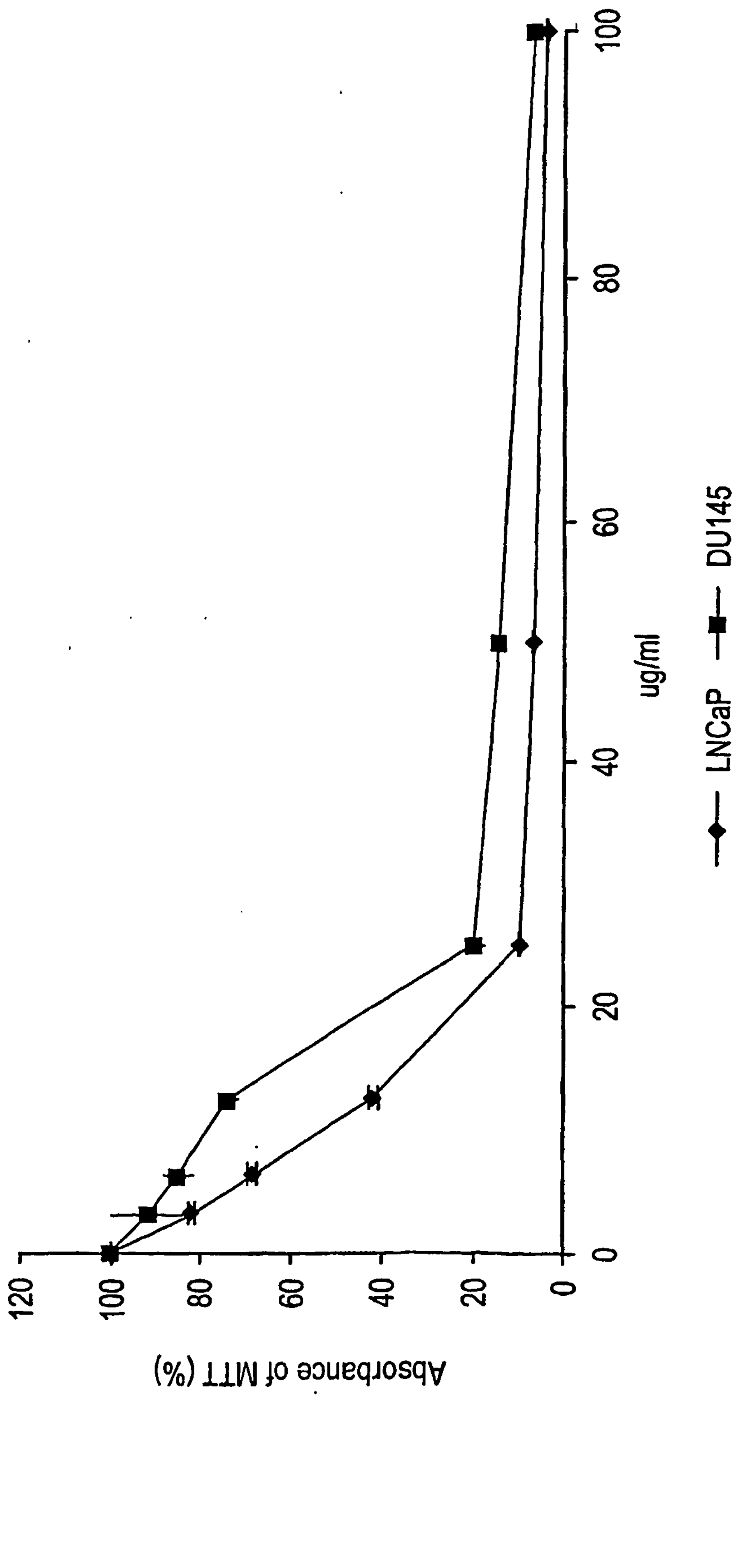

# FIG. 9

Cell viability (MTT) curves for Isoliquirtigenin treatment of LNCaP, DU145 and MCF-7

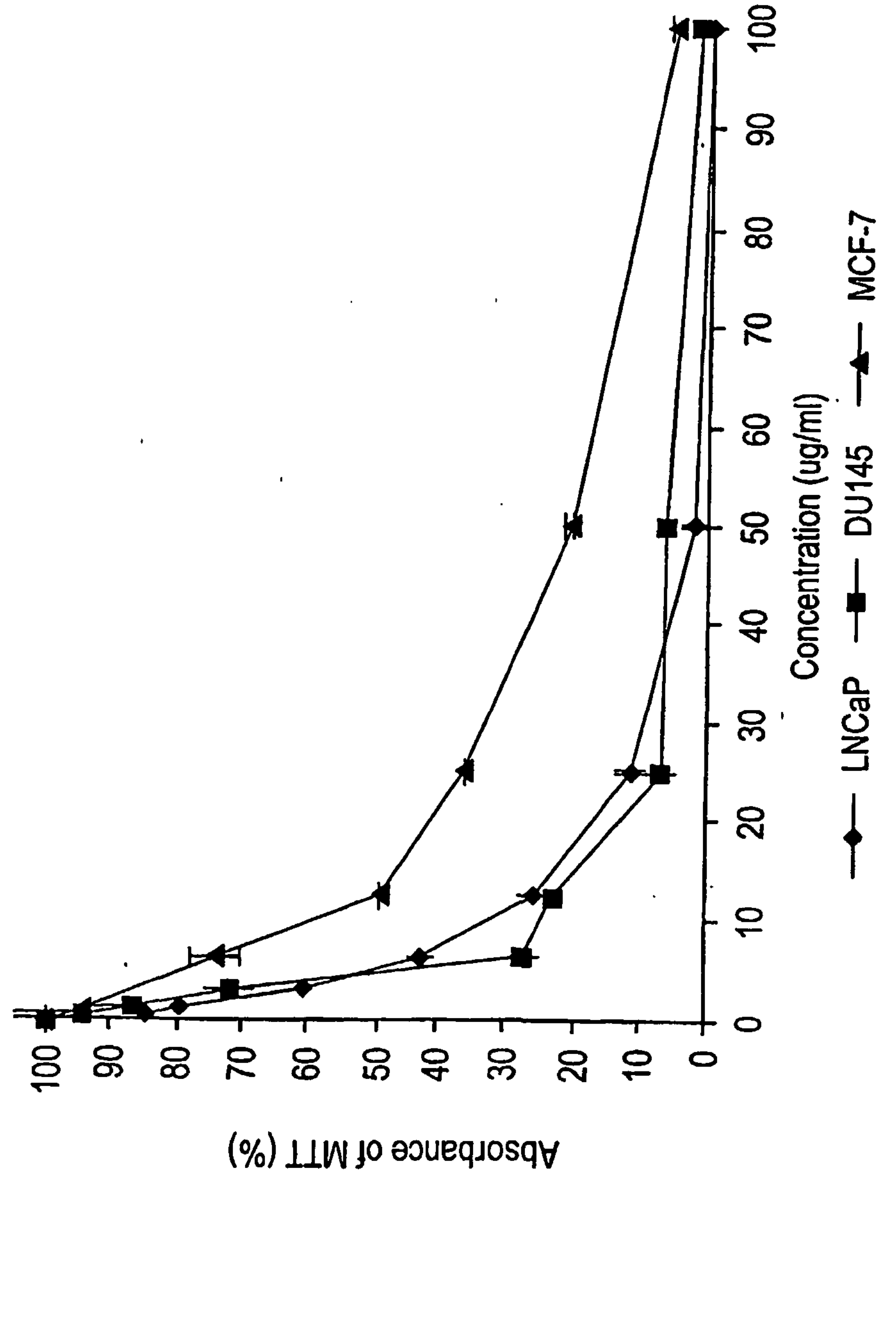

FIG. 10A

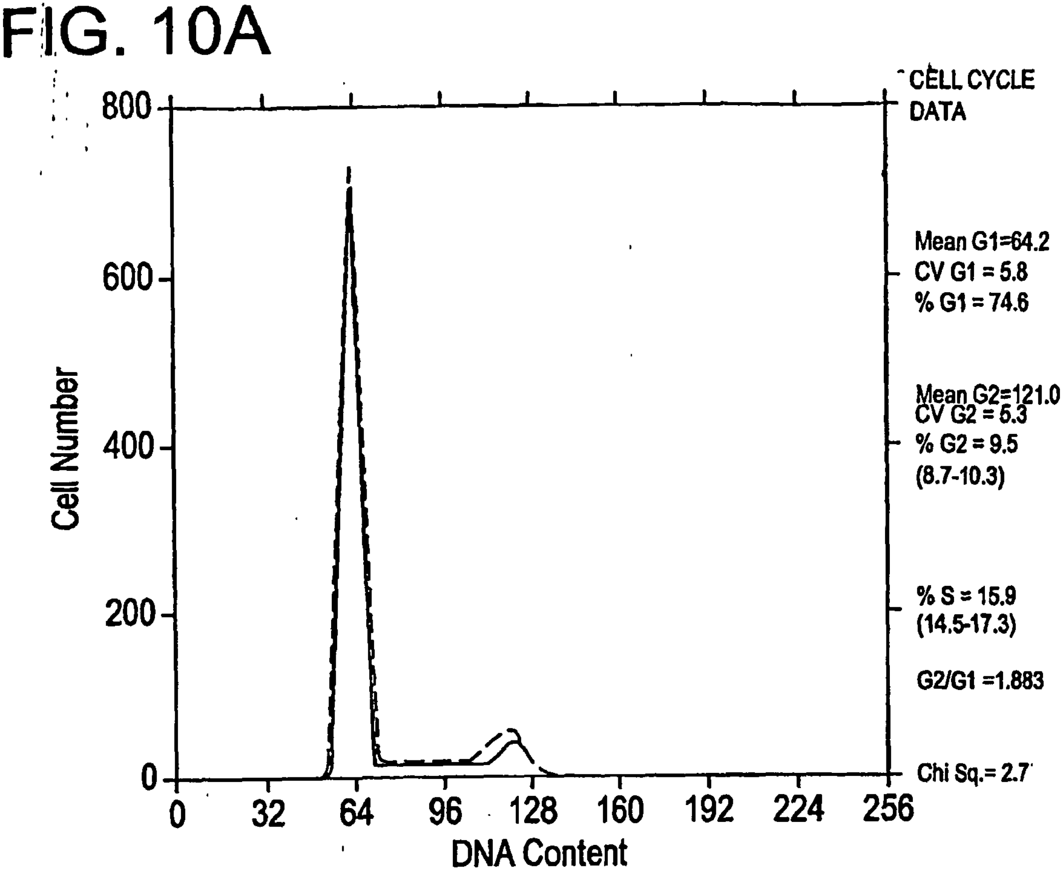


FIG. 10B

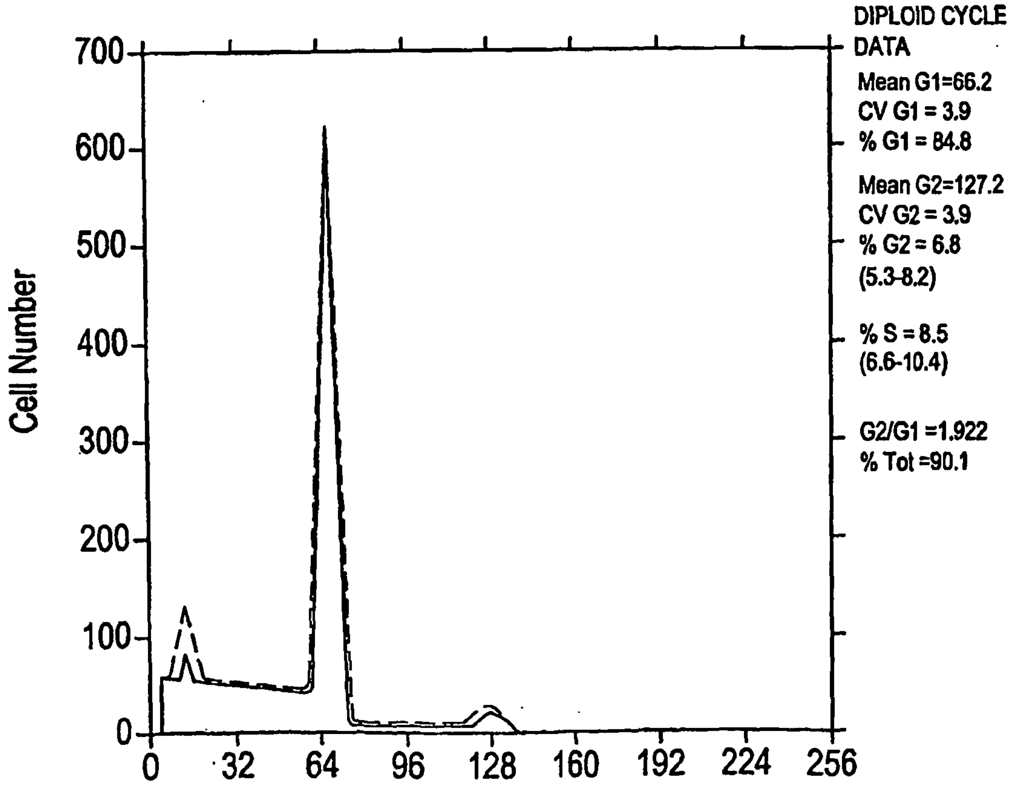

# FIG. 11

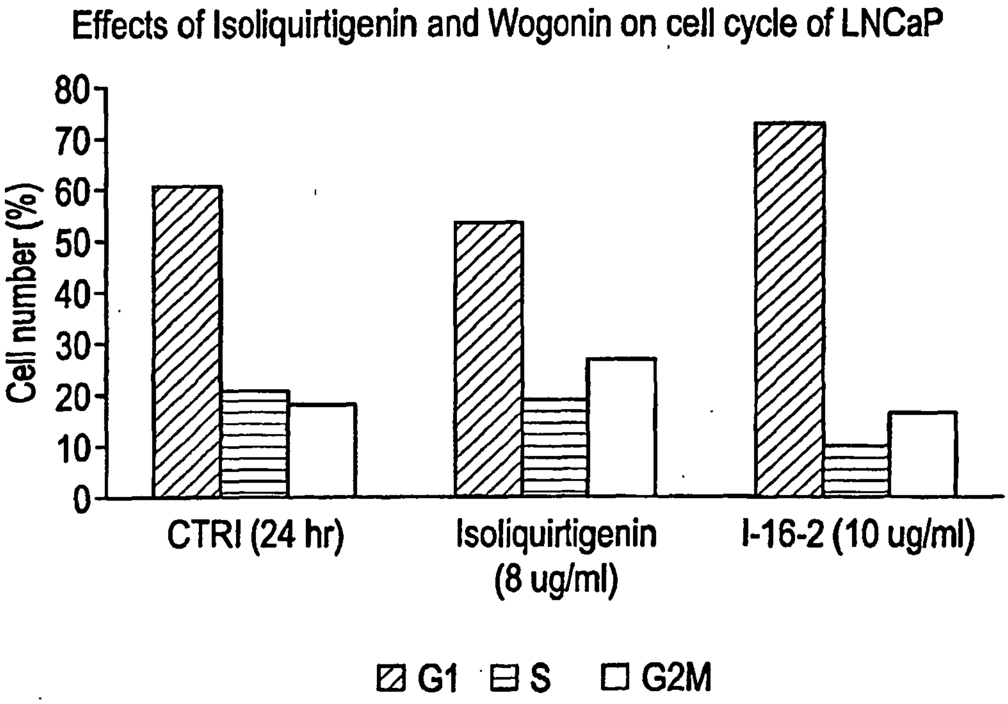
Effects of Isoliquirtigenin and Wogonin on cell cycle of LNCaP
Cell number (%)
80
70
60
50
40
30
20
10
0
CTRI (24 hr)
Isoliquirtigenin (8 ug/ml)
I-16-2 (10 ug/ml)
G1
S
G2M

FIG. 12

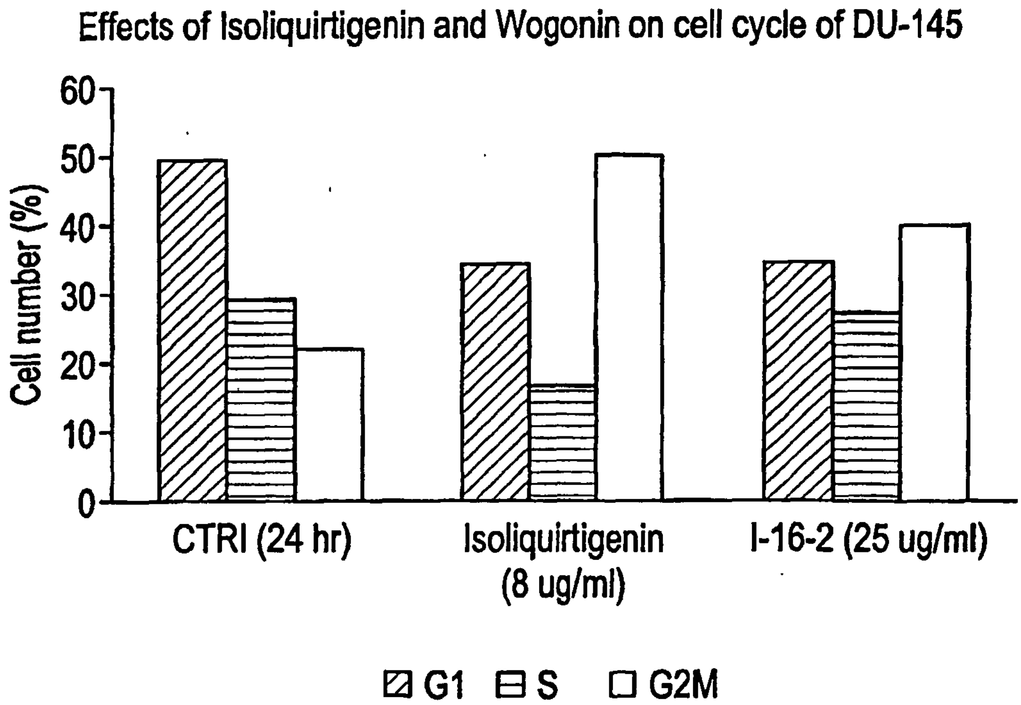
Effects of Isoliquirtigenin and Wogonin on cell cycle of DU-145
Cell number (%)
0
10
20
30
40
50
60
CTRI (24 hr)
Isoliquirtigenin (8 ug/ml)
I-16-2 (25 ug/ml)
G1
S
G2M

FIG. 13

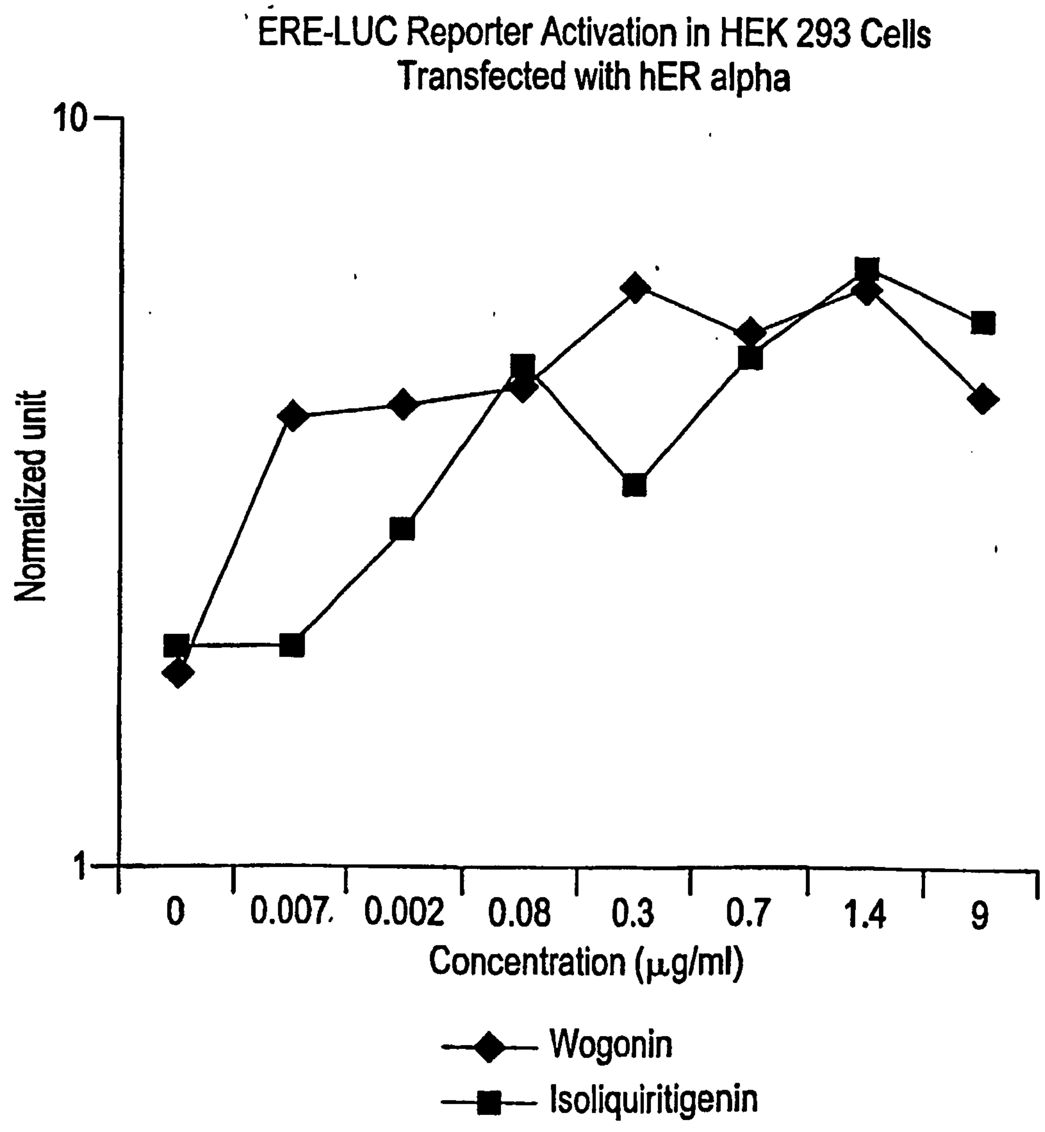
ERE-LUC Reporter Activation in HEK 293 Cells
Transfected with hER alpha
10
1
Normalized unit
0
0.007
0.002
0.08
0.3
0.7
1.4
9
Concentration (μg/ml)
Wogonin
Isoliquiritigenin

FIG. 14

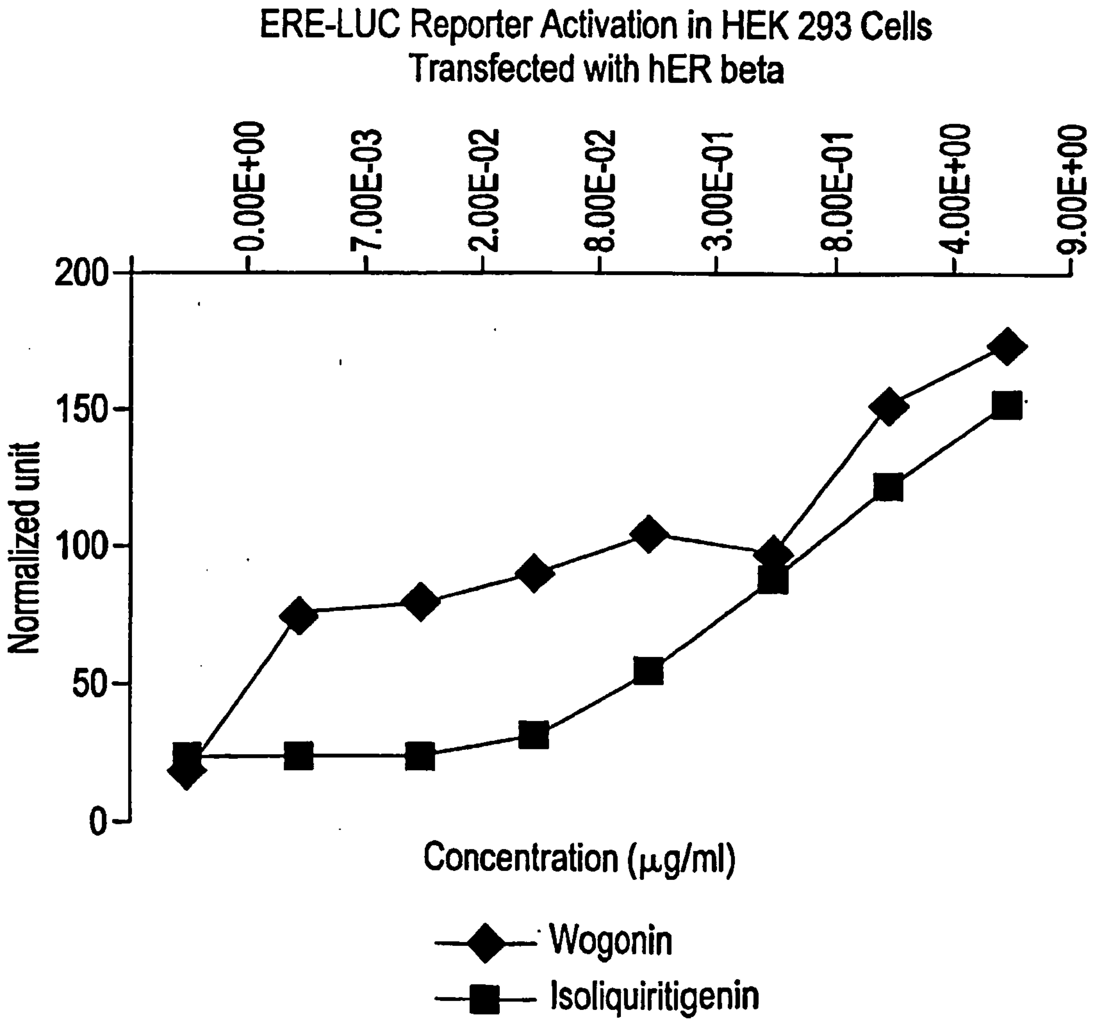
ERE-LUC Reporter Activation in HEK 293 Cells
Transfected with hER beta
0.00E+00
7.00E-03
2.00E-02
8.00E-02
3.00E-01
8.00E-01
4.00E+00
9.00E+00
200
150
100
50
0
Normalized unit
Concentration (µg/ml)
Wogonin
Isoliquiritigenin

FIG. 15

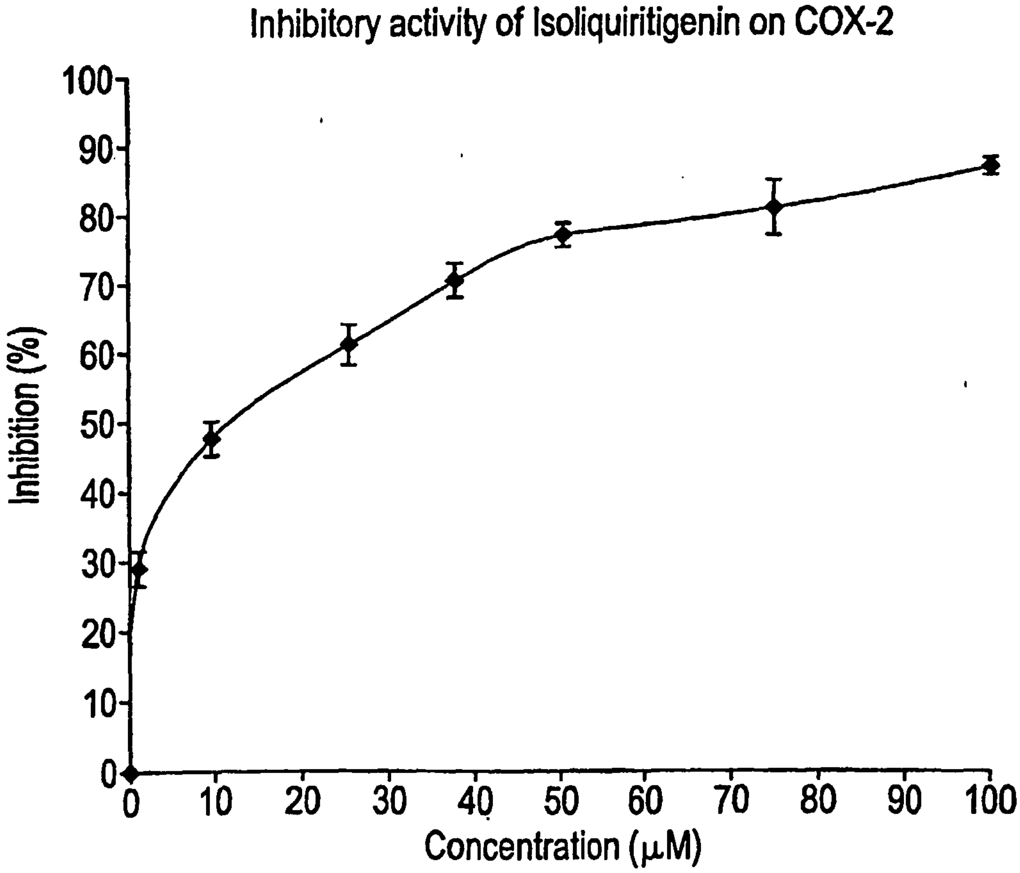
Inhibitory activity of Isoliquiritigenin on COX-2
Inhibition (%)
0
10
20
30
40
50
60
70
80
90
100
0
10
20
30
40
50
60
70
80
90
100
Concentration (μM)

FIG. 16

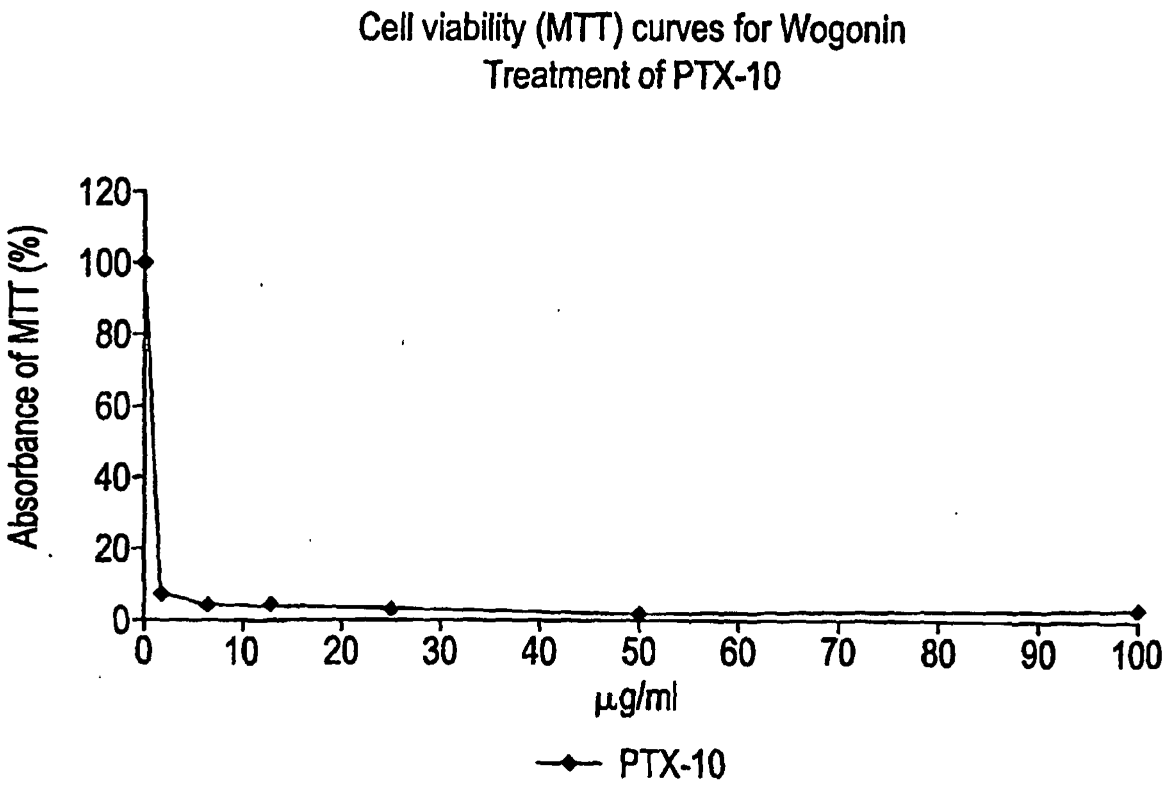

Cell viability (MTT) curves for Wogonin
Treatment of PTX-10
Absorbance of MTT (%)
120
100
80
60
40
20
0
0
10
20
30
40
50
60
70
80
90
100
μg/ml
PTX-10

FIG. 17

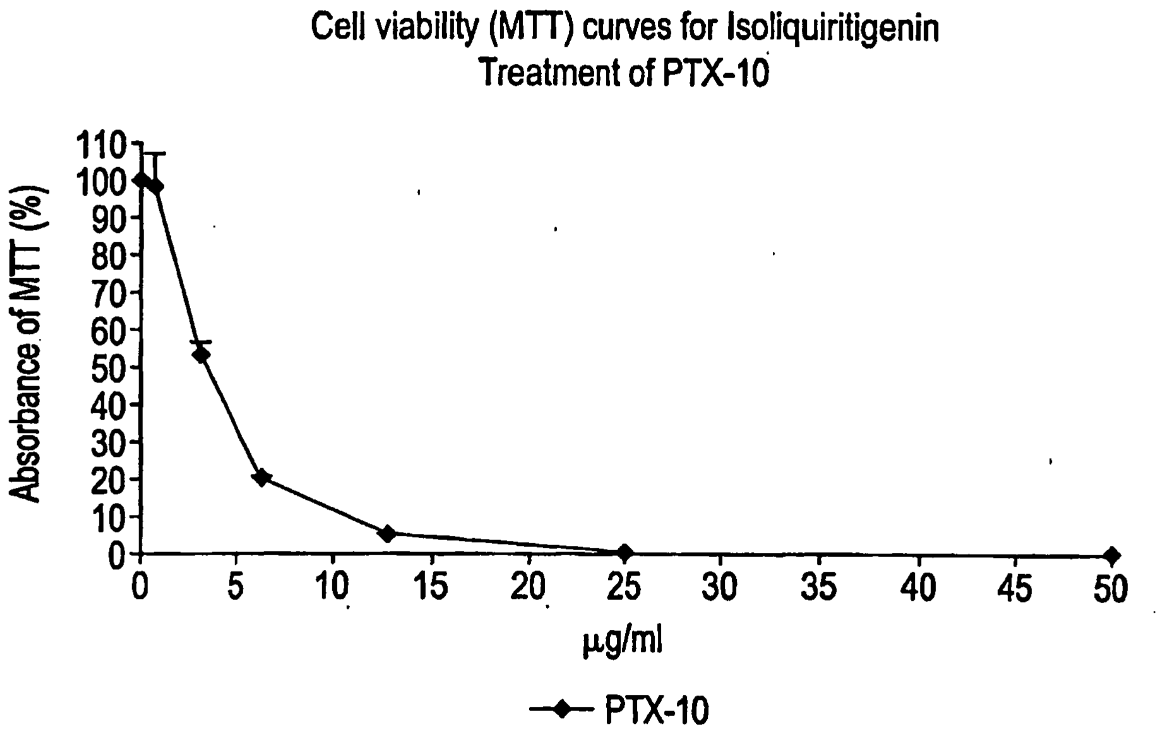
Cell viability (MTT) curves for Isoliquiritigenin
Treatment of PTX-10
Absorbance of MTT (%)
110
100
90
80
70
60
50
40
30
20
10
0
0
5
10
15
20
25
30
35
40
45
50
μg/ml
PTX-10